# EUROPEAN PATENT SPECIFICATION

(11) **EP 0 897 700 B1**
(45) Date of publication and mention of the grant of the patent: **17.07.2002**
(21) Application number: 98301613.0
(22) Date of filing: 04.03.1998
(51) Int. Cl.: A61F 2/06

(54) **Stent delivery assembly for treating bifurcations**
Vorrichtung zum Einführen eines Stents zur Behandlung von Verzweigungen
Appareil pour l'introduction d'un stent pour traiter des bifurcations

(30) Priority: 13.08.1997 US 910857
(43) Date of publication of application: 24.02.1999
(60) Divisional application: 01130605.7
(73) Proprietor: Advanced Cardiovascular Systems, Inc., Santa Clara, CA 95054-8167 (US)
(72) Inventor: Wilson, W. Stan, Missoula, Montana 59802 (US); Mauch, Kevin M., San Jose, California 95117 (US)
(74) Representative: McLeish, Nicholas Alistair Maxwell

(56) References cited:
- EP-A- 0 804 907
- WO-A-96/34580
- WO-A-97/41803
- WO-A-97/45073
- FR-A- 2 737 969

## Description

### BACKGROUND OF THE INVENTION

### Field of the Invention

The invention relates to stent deployment assemblies for use at a bifurcation and, more particularly, to a catheter assembly for implanting one or more stents for repairing bifurcations, the aorto-ostium, and bifurcated blood vessels that are diseased, as specified in the preamble of Claim 1. Such a stent delivery assembly is known e.g. from WO-A-96/34580.

### Prior Art

Stents conventionally repair blood vessels that are diseased and generally are hollow and cylindrical in shape, having terminal ends that are generally perpendicular to its longitudinal axis. In use, the conventional stent is positioned at the diseased area of a vessel and, after placement, the stent provides an unobstructed pathway for blood flow.

Repair of vessels that are diseased at a bifurcation is particularly challenging as the stent must overlay the entire diseased area at the bifurcation, yet not itself compromise blood flow. Therefore, the stent, without compromising blood flow, must overlay the entire circumference of the ostium to a diseased portion and extend to a point within and beyond the diseased portion. When the stent does not overlay the entire circumference of the ostium to the diseased portion, the stent fails to completely repair the bifurcated vessel. When the stent overlays the entire circumference of the ostium to the diseased portion, yet extends into the junction comprising the bifurcation, the diseased area is repaired, but blood flow may be compromised in other portions of the bifurcation. Unapposed stent elements may promote lumen compromise during neointimalization and healing, producing restenosis and requiring further procedures. Moreover, by extending into the junction comprising the bifurcation, the stent may block access to portions of the bifurcated vessel that require performance of further interventional procedures. Similar problems are encountered when a vessel is diseased at the point of its angled origin from the aorta, as in the ostium of a right coronary artery or a vein graft. In this circumstance, a stent overlying the entire circumference of the ostium extends back into the aorta, creating problems, including that of complicating catheter access to the vessel in any additional interventional procedures.

Conventional stents are designed to repair areas of blood vessels that are removed from the sites of bifurcations and, because a conventional stent generally terminates at right angles to its longitudinal axis, the use of conventional stents in the region of a vessel bifurcation may result in blocking the flow of blood through a side branch or may fail to repair the bifurcation to the full extent necessary. A conventional stent might be placed so that a portion of the stent extends into the pathway of blood flow to a side branch of the bifurcation or so that it extends so far as to completely cover the path of blood flow in a side branch. A conventional stent alternatively might be placed proximal to, but not entirely overlaying, the circumference of the ostium to the diseased portion. Such positioning of a conventional stent results in a bifurcation that is not completely repaired. The only conceivable situation in which a conventional stent having right-angled terminal ends could be placed where the entire circumference of the ostium is repaired without compromising blood flow, is where the bifurcation is formed of right angles. In such scenarios, extremely precise positioning of the conventional stent is required. This extremely precise positioning of the conventional stent may result in the right-angled terminal ends of the conventional stent overlying the entire circumference of the ostium to the diseased portion without extending into a side branch, thereby completely repairing the right-angled bifurcation.

To circumvent or overcome the problems and limitations associated with conventional stents in the context of repairing diseased bifurcated vessels, a stent that consistently overlays the entire circumference of the ostium to a diseased portion, yet does not extend into the junction comprising the bifurcation, may be employed. Such a stent has the advantage of completely repairing the vessel at the bifurcation without obstructing blood flow in other portions of the bifurcation. In addition, such a stent allows access to all portions of the bifurcated vessel, should further interventional treatment be necessary. In a situation involving disease located at the origin of an angulated aorto-ostial vessel, such a stent would have the advantage of completely repairing the vessel at the origin without protruding into the aorta and without complicating repeat access.

In addition to the problems encountered by using the prior art stents to treat bifurcations, the delivery platform for implanting such stents has presented numerous problems. For example, a conventional stent is implanted in the main vessel so that a portion of the stent is across the side branch, so that stenting of the side branch must occur through the main-vessel stent struts. In this method, commonly referred to in the art as the "monoclonal antibody" approach, the main-vessel stent struts must be spread apart to form an opening to the side-branch vessel and then a catheter with a stent is delivered through the opening. The "cell" or the portion of the main-vessel stent struts to be spread apart must be randomly and blindly selected by re-crossing the deployed stent with a wire. A drawback with this approach is that there is no way to determine or guarantee that the main-vessel stent struts are oriented properly with respect to the side branch or that the appropriate cell has been selected by the wire for dilatation. The aperture created often does not provide a clear opening and creates significant distortion in the surrounding stent struts. Thus, there is no way to tell if the main-vessel stent struts have been properly oriented and spread apart to provide a clear opening for stenting the side-branch vessel.

In another prior art method for treating bifurcated vessels, commonly referred to as the "Culotte technique," the side-branch vessel is first stented so that the stent protrudes into the main vessel. A dilatation is then performed in the main vessel to open and stretch the stent struts extending across the lumen from the side-branch vessel. Thereafter, the main-vessel stent is implanted so that its proximal end overlaps with the side-branch vessel. One of the drawbacks of this approach is that the orientation of the stent elements protruding from the side-branch vessel into the main vessel is completely random. Furthermore, the deployed stent must be re-crossed with a wire blindly, and then arbitrarily selecting a particular stent cell. When dilating the main vessel, the stretching of the stent struts therefore is random, leaving the possibility of restricted access, incomplete lumen dilatation, and major stent distortion.

In another prior art device and method of implanting stents, a "T" stent procedure includes implanting a stent in the side-branch ostium of the bifurcation followed by stenting the main vessel across the side-branch ostium. In another prior art procedure, known as "kissing" stents, a stent is implanted in the main vessel with a side-branch stent partially extending into the main vessel creating a double-barreled lumen of the two stents in the main vessel distal to the bifurcation. Another prior art approach includes a so-called "trouser legs and seat" approach, which includes implanting three stents, one stent in the side-branch vessel, a second stent in a distal portion of the main vessel, and a third stent, or a proximal stent, in the main vessel just proximal to the bifurcation.

All of the foregoing stent deployment assemblies suffer from the same problems and limitations. Typically, there are uncovered segments of the intimal surface of the main vessel and side-branch vessels between the segments that are stented. An uncovered flap or fold in the intima or plaque will invite a "snowplow" effect, representing a substantial risk for sub-acute thrombosis, and the increased risk of restenosis. Further, when portions of the stent are left un-apposed within the lumen, the risk of sub-acute thrombosis or restenosis again is increased. The prior art stents and delivery assemblies for treating bifurcations are difficult to use, making successful placement of the stents nearly impossible. Further, even where placement has been successful, the side-branch vessel can be "jailed" or covered so that there is impaired access to the stented area for subsequent intervention.

In an attempt to overcome these difficulties there is disclosed in WO 96/34580 (which corresponds to the preamble of Claim 1) an endoprosthesis for the treatment of blood-vessel bifurcation stenosis that is characterised by the fact that it comprises three tubular sections and two connectors. These sections comprise a proximal section and a first distal section aligned at least approximately with the proximal section and intended for insertion into a first blood vessel branching off on the bifurcation. The first distal section is linked to the proximal section by a first lateral connector. A second distal section is located at the side of the first distal section and intended for insertion into a second vessel branching off from the bifurcation. The two distal sections have their proximal ends linked by a second connector. There is also described a double-balloon dilation system.

WO 97/45073 represents prior art by virtue of A54(3) EPC and describes a bifurcated endovascular stent formed from a pair of individual tubular component stents that can be placed independently in sequence in the branched region of a body vessel to form the bifurcated stent in situ. The first placed component stent includes a side opening and may be placed to extend both proximally and distally of the bifurcated junction with the side opening facing the entry to the branch vessel. The first component stent then is expanded within the vessel. The second component stent then can be advanced through the first component stent, through the side opening and into the branch vessel where it can be expanded. The portions of the first and second component stents that surround the region of the vessel juncture are constructed to provide enhanced lateral support for the walls of the vessel in the region of the junction. A delivery catheter is provided and includes a guide wire duct adapted to facilitate entry of a guide wire into the branch vessel while the first component stent is deployed in the other lumen of the vessel.

In addition to the problems that are encountered in treating disease involving bifurcations for vessel origins, difficulty also is encountered in treating disease that is confined to a vessel segment which extends very close to a distal branch point or bifurcation that is not diseased and that does not require treatment. In such circumstances, it may be difficult or impossible to very precisely place a stent to cover the distal segment, but not to extend into the ostium of the distal side-branch.

Preferred embodiments of the present invention offer a solution to the above problems.

References to distal and proximal herein shall mean as follows: the proximal direction is moving away from or out of the patient and the distal direction is moving toward or into the patient. These definitions will apply with reference to body lumens and apparatus, such as catheters, guide wires, and stents.

### SUMMARY OF THE INVENTION

Preferred embodiments of the present invention provide improved stent delivery assemblies for repairing a main vessel and a side-branch vessel forming a bifurcation, without compromising blood flow in other portions of the bifurcation, thereby allowing access to all portions of the bifurcated vessels should further interventional treatment be necessary. In addition, they provide an improved stent delivery system for repairing disease which confines the stent to the aorto-ostium of a vessel without protrusion into the aorta.

There is also described a proximal-angled stent for implanting in a side-branch vessel adjacent to a bifurcation. The cylindrical member of the stent can have substantially any outer wall surface typical of conventional stents which have been used, for example, in the coronary arteries. The cylindrical member of the proximal-angled stent has a distal end forming a first plane section that is substantially transverse to the longitudinal axis of the stent. The proximal end of the stent forms a second plane section that is at an angle, preferably an acute angle, measured relative to the longitudinal axis of the stent. The acute angle is selected to approximately coincide with the angle formed by the intersection of the side-branch vessel and the main vessel, so that after deployment no portion of the stented area in the side-branch vessel is left uncovered, and no portion of the proximal-angled stent extends into the main vessel.

A second stent is described for implanting in the main vessel adjacent to a bifurcation having a cylindrical member with distal and proximal ends and an outer wall surface therebetween, which typically can be similar to the outer wall surface of stents that have been used in the coronary arteries. An aperture is formed in the outer wall surface of the second stent and is sized and positioned on the outer wall surface so that when the apertured stent is implanted in the main vessel, the aperture is aligned with the side-branch vessel and the proximal-angled stent in the side-branch vessel, providing unrestricted blood flow from the main vessel through to the side-branch vessel. Deployment of the angled and apertured stents is accomplished by a stent delivery system adapted specifically for treating bifurcated vessels.

According to the present invention there is provided a stent delivery assembly for treating bifurcated vessels having a side-branch vessel and a main vessel, comprising: a side-branch catheter having a proximal end and a distal end; an expandable member proximate the distal end of the catheter; a tracking guide wire lumen extending within at least a portion of the catheter; a tracking guide wire having a distal end and a proximal end and sized for slidable movement within the tracking guide wire lumen; and a positioning guide wire lumen having an angulated section and being firmly attached to an outer surface of the catheter and associated with the expandable member in both its unexpanded and fully expanded condition and adapted to receive for slidable engagement a positioning guide wire, the positioning guide wire having a distal end and a proximal end, the proximal ends of the tracking and positioning guide wires extending out of the patient and manipulatable so that the distal end of the positioning guide wire is advanced in the main vessel distal to the side-branch vessel, and the distal end of the tracking guide wire is advanced into the side-branch vessel.

The stent positioning guide wire lumen includes an angulated section so that the stent-positioning guide wire advanced in the main vessel distal to the side-branch vessel results in rotation, causing the proximal-angled stent to assume the correct position in the side-branch vessel. The positioning lumen functions to orient the stent-positioning guide wire to rotate or to torque the side-branch catheter so as to properly align and position the proximal-angled stent in the side-branch vessel.

The side-branch catheter assembly is capable of delivering the proximal-angled stent, mounted on the expandable member, in the side-branch vessel. The side-branch catheter also could be configured for delivering a self-expanding proximal-angled stent.

The stent delivery system may further include a main-vessel catheter for delivering a stent in the main vessel after the side-branch vessel has been stented. In a preferred embodiment the main-vessel catheter includes a tracking guide wire lumen, extending through at least a portion thereof, which is adapted for receiving a tracking guide wire for slidable movement therein. An expandable member is positioned near the main-vessel catheter distal end for delivering and implanting a main-vessel (apertured) stent in the main vessel. The main-vessel stent includes an aperture on its outer surface which aligns with the side-branch vessel. A positioning guide wire lumen is associated with the expandable member, and is sized for slidably receiving the stent-positioning guide wire. The stent-positioning guide wire slides within the positioning guide wire lumen to orient the expandable member so that it is positioned adjacent to, but not in, the side-branch vessel with the aperture of the stent facing the side-branch ostium.

In a preferred embodiment, both the side-branch catheter and main-vessel catheter assemblies include the so-called rapid exchange catheter features, which allow one catheter to be exchanged easily for another catheter while the tracking and positioning guide wires remain positioned, respectively, in the side-branch vessel and the main vessel. In an alternate embodiment, both catheters may be of the "over-the-wire" type.

In addition to the above there is also described a method for delivering the proximal-angled and the main-vessel (apertured) stents in the bifurcated vessel. In a preferred arrangement of the side-branch catheter system (i.e., side-branch catheter and proximal-angled stent), the distal end of the tracking guide wire is advanced into the side-branch vessel and distally of the target area. The side-branch catheter then is advanced along the tracking guide wire until the distal end of the catheter is just proximal of the side-branch. The distal end ofthe integrated, stent-positioning guide wire then is advanced by the physician, who pushes the guide wire from outside the body. The distal end of the stent-positioning wire travels through the positioning guide wire lumen, passes close to the proximal end of the proximal-angled stent and expandable member, and then exits the lumen. The wire is advanced in the main vessel until the distal end is distal to the side-branch vessel. The catheter then is advanced into the side branch until resistance is felt, owing to the stent-positioning guide wire pushing up against the ostium of the side-branch vessel, which causes the proximal-angled stent to rotate into position and to arrest its advancement at the ostium. Thereafter, the proximal-angled stent, mounted on the expandable member, is aligned across the side-branch vessel target area and the angled proximal end of the stent is aligned at the intersection of the side-branch vessel and the main vessel (the ostium of the side-branch vessel) so that the stent completely covers the target area in the side-branch vessel, but does not extend into the main vessel, where it may block blood flow. The expandable member is expanded thereby expanding and implanting the proximal-angled stent in the side-branch vessel. The positioning wire prevents forward movement ofthe expandable member and proximal-angled stent during inflation. Thereafter, the expandable member is deflated and the side-branch catheter assembly is withdrawn from the patient in a known rapid-exchange manner. In this arrangement, the side-branch catheter is designed so that both the side-branch tracking guide wire and main-vessel positioning guide wire can be left in the vessel in which each respectively was deployed in the event sequential or simultaneous high pressure balloon inflation is required in a vessel in order to complete the stenting procedure. In other words, the integrated positioning wire can be unzipped from the proximal 100 cm of the catheter thereby allowing it to act as a rapid exchange wire. Preferably, high pressure balloons are inflated simultaneously in the main vessel stent and proximal-angled stent, in order to avoid deforming one stent by un-opposed balloon inflation within the other one. This additional step of high pressure balloon inflation is a matter of physician preference. A further advantage of this arrangement is that by refraining from advancing the integrated stent-positioning wire out of catheter until the catheter distal end is near the target area avoids wire wrapping, which can be encountered in an arrangement using two non-integrated guide wires. When using this method, the side-branch vessel can be stented without the need for stenting the main vessel.

In an aorto-ostial application of the side-branch catheter assembly (side-branch catheter together with a proximal angulated stent), the positioning wire is advanced into the aortic root while the tracking wire is advanced into the right coronary or vein graft, the angulated origin of which is to be stented. After the proximal-angled stent mounted on the expanding member is advanced, it is aligned across the target area and the angled proximal end of the stent is aligned at the ostium.

In the event the main vessel is to be stented (with the stent placed across the bifurcation site), the proximal end of the main-vessel guide wire is inserted into the distal end of the guide wire lumen of the main-vessel catheter. The side-branch wire would be removed from the side branch at this time. The main-vessel catheter then would be advanced into the body until the catheter is within 1 cm or so of the target site. The distal end of the second (integrated, stent-positioning) guide wire, which resides in the main-vessel catheter during delivery to the main vessel, then is advanced by having the physician push the positioning wire from outside the body. The distal end of the stent-positioning wire travels through the positioning guide wire lumen and passes underneath the proximal half of the stent until it exits at the site of the stent aperture or at the location of a designated stent cell where an aperture can be formed. The catheter then is advanced distally until resistance is felt, owing to the stent-positioning guide wire pushing up against the ostium of the side-branch vessel, which indicate that the stent aperture correctly is facing the side-branch vessel ostium and is aligned with the proximal end of the proximal-angled stent. Thereafter, the expandable member on the main-vessel catheter is inflated, thereby expanding the main-vessel stent into contact with the main vessel and implanting it, with the aperture in the stent providing an unobstructed flow path for the blood from the main vessel through to the side-branch vessel. The expandable member then is deflated and the main-vessel catheter is removed from the body. The main-vessel catheter is designed so that both the main-vessel guide wire and side-branch wire can be left in the vessels in which each respectively was deployed in the event sequential or simultaneous high pressure balloon inflation is required in a vessel in order to complete the stenting procedure. The presence of the stent-positioning wire in the stent aperture permits catheter access through this aperture into the side-branch vessel for balloon inflation to smooth out the aperture in the main-vessel stent. This additional step is a matter of physician preference.

When using this method, the main vessel can be stented without the need for stenting the side-branch vessel. An advantage of this arrangement is that a major side branch, not diseased and not requiring treatment, that exits off from a main vessel that does require stenting, may be protected by the positioning wire while the main vessel is stented. If "snowplowing" compromise or closure of the side-branch vessel should occur in the course of the main-vessel stenting, then access already is established and guaranteed for stenting of the side-branch vessel over the wire already in place in the manner described above. This will allow confident stenting of a main vessel segment containing a major side branch. In this usage, additional stenting of the side branch will be required only if compromise or occlusion of the side branch occurs.

In an alternative arrangement, a main-vessel stent that does not have an aperture on its outer surface is mounted on the main-vessel catheter and is implanted in the main vessel so that it spans the opening to the side-branch vessel. Thereafter, a balloon catheter is inserted through a targeted (non-random) stent cell ofthe main-vessel stent, which is centered precisely facing the side-branch ostium, so that the balloon partially extends into the side-branch vessel. This balloon has tracked over the positioning wire which has been left in place through the targeted stent cell during and after deployment of the main vessel stent. The balloon is expanded, forming an opening through the stent struts that corresponds to the opening of the side-branch vessel, providing a blood-flow path through the main vessel and main-vessel stent and into the side-branch vessel. A proximal-angled stent mounted on a side-branch catheter then is advanced through the main-vessel stent and the opening formed in the targeted stent cell through to the side-branch vessel. The proximal-angled stent is expanded and implanted in the side-branch vessel so that all portions of the side-branch vessel are covered by the stent in the area of the bifurcation. After the main-vessel stent and the side-branch vessel proximal-angled stent are implanted, an uncompromised blood-flow path is formed from the main vessel through the main-vessel stent and opening into the side-branch vessel, and through the side-branch vessel proximal-angled stent.

In another arrangement, a stent having a distal angle is implanted in the main vessel. In portions of the main vessel having disease that approach and are directly adjacent to the side-branch vessel, a distal angle stent is implanted using the catheter so that the stent covers the diseased areas, but does not jail or cover the opening to the side-branch vessel.

In another arrangement, a Y-shaped catheter and Y-shaped stent are provided for stenting a bifurcated vessel. In this arrangement, a dual balloon catheter has a Y-shaped stent mounted on the balloons and the balloons are positioned side by side for easier delivery. The balloons normally are biased apart, but are restrained and held together to provide a low profile during delivery of the stent. A guide wire first is positioned in a main vessel at a point distal to the bifurcation. A second guide wire is retained in the catheter in a second guide wire lumen while the catheter is advanced over the tracking guide wire so that the balloons and stent are distal to the bifurcation. The tracking guide wire then is withdrawn proximally thereby releasing the balloons which spring apart. The catheter is withdrawn proximally until it is proximal to the bifurcation. As the catheter is withdrawn proximally, one of the two guide wires is left in the main vessel. The other guide wire then is advanced into the side-branch vessel. The catheter then is advanced over both guide wires until the balloons and stent are anchored in the bifurcation. The balloons are inflated and the stent is expanded and implanted in the bifurcation.

In another arrangement, two apertured stents are implanted to cover the bifurcated vessels. A main-vessel stent has a cylindrical shape having a heavy cell density on the distal half and a more sparse or "light" cell density on the proximal half, and an aperture on the outer surface at the junction at these two halves. A main-vessel stent first is implanted in the main vessel so that its aperture aligns with the ostium of the side-branch vessel, thereby covering the main vessel proximally with light cell density and distally with heavy cell density. A second main-vessel stent then is implanted over a tracking wire into the side branch so that the heavy cell density portion of the stent is implanted in the side-branch vessel, the light cell density is implanted in the main vessel and overlaps the light cell density of the proximal end of the main-vessel stent, and the aperture faces the main vessel as it departs from the side branch. Combined densities of proximal light cell portions proximal to the bifurcation are similar to the heavy cell densities in each limb distal to the bifurcation. Respective apertures of each of the two main-vessel stents are aligned with the respective ostia of both limbs of the bifurcation (main vessel and side branch).

Other features and advantages of the present invention will become apparent from the following detailed description, taken in conjunction with the accompanying drawings, which illustrate, by way of example, the principles of the invention.

### BRIEF DESCRIPTION OF THE DRAWINGS

FIGURE 1 is an elevational view of a bifurcation in which a prior art "T" stent is in a side-branch ostium followed by the stenting ofthe main vessel across the branch ostium.

FIG. 2 is an elevational view of a bifurcation in which "touching" prior art stents are depicted in which one stent is implanted in the side branch, a second stent 18 implanted in a proximal portion of the main vessel next to the branch stent, with interrupted placement of a third stent, implanted more distally in the main vessel.

FIG. 3 is an elevational view of a bifurcation depicting "kissing" stents where a portion of one stent is implanted in both the side-branch and the main vessel and adjacent to a second stent implanted in the main vessel, creating a double-barreled lumen in the main vessel distal to the bifurcation.

FIG. 4 is an elevational view of a prior art "trouser legs and seat" stenting approach depicting one stent implanted in the side-branch vessel, a second stent implanted in a proximal portion of the main vessel, and a close deployment of a third stent distal to the bifurcation leaving a small gap between the three stents of an uncovered luminal area.

FIG. 5A is a perspective view of a stent having an angled proximal end.

FIG. 5B is a side elevational view of the proximal-angled stent of FIG. 5A depicting the distal end being transverse to the longitudinal axis of the stent, and the proximal end at an angle of less than 90°.

FIG. 5C is an elevational view of a bifurcation in which a prior art stent is implanted in the side-branch vessel.

FIG. 5D is an elevational view of a bifurcation in which a prior art stent is implanted in the side-branch vessel, with the proximal end of the stent extending into the main vessel.

FIG. 5E is an elevational view of a bifurcation in which the proximal-angled stent depicted in FIGS. 5A and 5B, is implanted in the side-branch vessel.

FIG. 6A is a perspective view depicting a main-vessel stent in which an aperture is formed on the outer surface of at least a portion of the stent.

FIG. 6B is a side elevational view of the main-vessel stent of FIG. 6A.

FIG. 7A is an elevational view, partially in section, of a side-branch catheter assembly depicting the distal end of the catheter with the expandable member and the second guide wire lumen attached thereto, for receiving the integrated stent-positioning guide wire, while the tracking guide wire is received by the main guide wire lumen.

FIG. 7B is an elevational view, partially in section, of the catheter assembly of FIG. 7A, in which the stent positioning guide wire is advanced out of the catheter.

FIG. 8 is an elevational view, partially in section, of a side-branch catheter assembly depicting an expandable balloon having an angled proximal portion corresponding to the angle of the proximal-angled stent.

FIG. 9A is an elevational view of a bifurcated vessel in which a side-branch tracking guide wire has been advanced into a side-branch vessel, with the stent-positioning guide wire remaining within the catheter until the catheter assembly is just proximal to the side-branch vessel.

FIG. 9B is an elevational view of a bifurcation in which a side-branch tracking guide wire has been advanced through the patient's vascular system into a side branch, and a stent-positioning guide wire has been advanced through the patient's vascular system and into the main vessel distal to the ostium of the side-branch vessel.

FIG. 10A is an elevational view of a bifurcation in which the side-branch catheter assembly has been advanced in the patient's vasculature so that the proximal-angled stent mounted on the expandable member is positioned in the target area of the side-branch vessel.

FIG. 10B is an elevational view of the side-branch catheter assembly of FIG. 10A in which the proximal-angled stent has been expanded by the balloon portion of the catheter in the side-branch vessel.

FIGS. 11A-11D are partial elevational views in which the side-branch catheter assembly of FIG. 10A is used to implant the proximal-angled stent in the side-branch vessel where the proximal-angled stent is rotated to be properly aligned for implanting in the vessel.

FIGS. 12A-12C depict an elevational view, partially in section, ofa main-vessel catheter assembly in which the main vessel stent has an aperture on its outer surface.

FIGS. 12D-12F depict an elevational view, partially in section, of the main-vessel catheter of FIGS. 12A-12C with a ramp to help orient and advance the guide wire through the aperture in the main-vessel stent.

FIGS. 12G-12I depict an elevational view, partially in section, of an alternative to the main-vessel catheter of FIGS. 12A-12C in which the guide wire lumen is angled to pass under the stent and exit through the stent aperture.

FIGS. 12J-12L depict an elevational view, partially in section, of an alternative to the main-vessel catheter of FIGS. 12A-12C in which a portion of the guide wire lumen passes under the stent.

FIGS. 13A-13E are elevational views, partially in section, depicting the main-vessel catheter assembly of FIG. 12A and the main-vessel stent in which two guide wires are used to correctly position the main vessel stent so that the aperture in the stent is aligned with the side-branch vessel.

FIG. 14 is an elevational view of a bifurcated vessel in which the proximal-angled stent is implanted in the side-branch vessel and a main vessel stent is implanted in the main vessel.

FIG. 15 is a perspective view of a main-vessel stent for deployment in the main vessel, where a targeted stent cell provides an opening through which a guide wire can pass.

FIGS. 16A-16D are elevational views, partially in section, of a main vessel catheter having the main vessel stent of FIG. 15 mounted thereon, and its relationship to the guide wire for advancing through a targeted stent cell.

FIG. 17 is an elevational view of a bifurcation in which a main-vessel stent is positioned in a main vessel so that it spans the opening to the side-branch vessel.

FIG. 18 is an elevational view of a bifurcation in which a main-vessel stent is implanted in the main vessel and a balloon catheter is partially inserted into a side-branch vessel to form an opening through the targeted stent cell of the main stent.

FIGS. 19A-19C are elevational views of a bifurcation in which a main-vessel stent is first implanted in the main vessel and a catheter assembly next deploys a proximal-angled stent in a side-branch vessel.

FIGS. 19D and 19E are cross-sectional views looking down the side-branch vessel at an expanded main vessel prior art stent in which a random, sub-optimal stent cell was entered and expanded.

FIGS 19F is a cross-sectional view looking down the side-branch vessel at an expanded main-vessel stent in which a proper targeted stent cell was entered and expanded.

FIG. 20A is an elevational view, partially in section, depicting a main vessel catheter in which the main vessel stent is mounted over a positioning guide wire lumen.

FIG. 20B is an elevational view, partially in section, of a main vessel catheter depicting the main vessel stent mounted over a section of the positioning guide wire lumen, with a distal portion of the guide wire lumen associated with the distal tip of the catheter.

FIG. 20C is an elevational view, partially in section, of the catheter of FIG. 20B depicting the positioning guide wire advanced out of the positioning guide wire lumen.

FIG. 20D is an elevational view, partially in section, depicting a main-vessel stent implanted in the main vessel without jailing or covering the side-branch vessel.

FIG. 20E is an elevational view, partially in section, depicting the main-vessel catheter of FIG. 20A having a ramp to assist in positioning the guide wire.

FIG. 20F is an elevational view, partially in section, of a distal angled stent being implanted in the main vessel without jailing the side-branch vessel.

FIGS. 21 and 22 are elevational views, partially in section, depicting an alternative to the main-vessel catheter of FIG. 20B in which the distal end of the guide wire lumen springs away from the expandable balloon.

FIGS. 23A-23B, 24A-24B, 25A-25B, and 26A-26B, are elevational views of various bifurcations which are indicated for receiving main vessel and side-branch vessel stents deployed by the catheters described.

FIG. 27A is an elevational view, partially in section, depicting an arrangement in which a Y-shaped catheter assembly deploys a Y-shaped stent in the bifurcation.

FIG 27B is an elevational view depicting an arrangement in which a dual balloon catheter assembly deploys a Y-shaped stent in the bifurcation.

FIG. 28 is an elevational view depicting the Y-shaped catheter assembly of FIG. 27A in which the stent is mounted on the balloon portions of the catheter.

FIG. 29A is an elevational view, partially in section of a bifurcation in which the Y-shaped catheter of FIG. 27A is delivering the stent in the bifurcated area, tracking over the wire that joins the two tips together.

FIG. 29B is an elevational view, partially in section, of a bifurcation in which the delivered Y-shaped balloon components have been released and spread apart by withdrawal of the tracking wire from the other balloon tip lumen.

FIG. 29C is an elevational view, partially in section, of the Y-shaped delivery catheter of FIG. 27A in which the Y-shaped balloon has been withdrawn proximal to the bifurcation, leaving the first wire in the right branch.

FIG. 30 is an elevational view, partially in section, of the Y-shaped delivery catheter of FIG. 27A in which the second guide wire is advanced into the left branch.

FIG. 31 is an elevational view depicting the Y-shaped catheter of FIG. 27A in which the Y-shaped stent is implanted in the side branch and main vessels of the bifurcation.

FIG. 32 is an elevational view, partially in section, depicting the Y-shaped catheter assembly of FIG. 27A in which the Y-shaped stent has been implanted and the balloon portions of the catheter have been deflated.

FIG. 33 is an elevational view depicting a bifurcated vessel in which the catheter of FIG. 27A has been withdrawn after implanting the Y-shaped stent.

FIG. 34 is an elevational view depicting a modified stent having an aperture in its sidewall and in which half of the stent has a heavy stent cell density while the other half of the stent has a light stent cell density.

FIG. 35 is an elevational view depicting the stent of FIG. 35 combined to form a stent having a heavy stent cell density in all portions.

FIG. 36A is an elevational view depicting a bifurcation, in which the stent of FIG. 35 has been implanted so that the aperture corresponds to the side-branch vessel and the stent is implanted in the main vessel.

FIG. 36B is an elevational view depicting a bifurcating vessel in which the stent of FIG. 34 has been implanted so that the heavy stent cell density is in the side-branch vessel and the light cell density is in the main vessel. The aperture corresponds to the continuing lumen of the main vessel.

FIG. 36C is an elevational view depicting a bifurcated vessel in which two stents of FIG. 34 have been implanted in the side-branch vessel and the main vessel respectively so that the light stent cell density of each overlaps with the light cell density of the other thereby creating cell density proximal to the bifurcation similar to the heavy cell density present in each limb distal to the bifurcation.

### DETAILED DESCRIPTION OF THE PREFERRED EMBODIMENTS

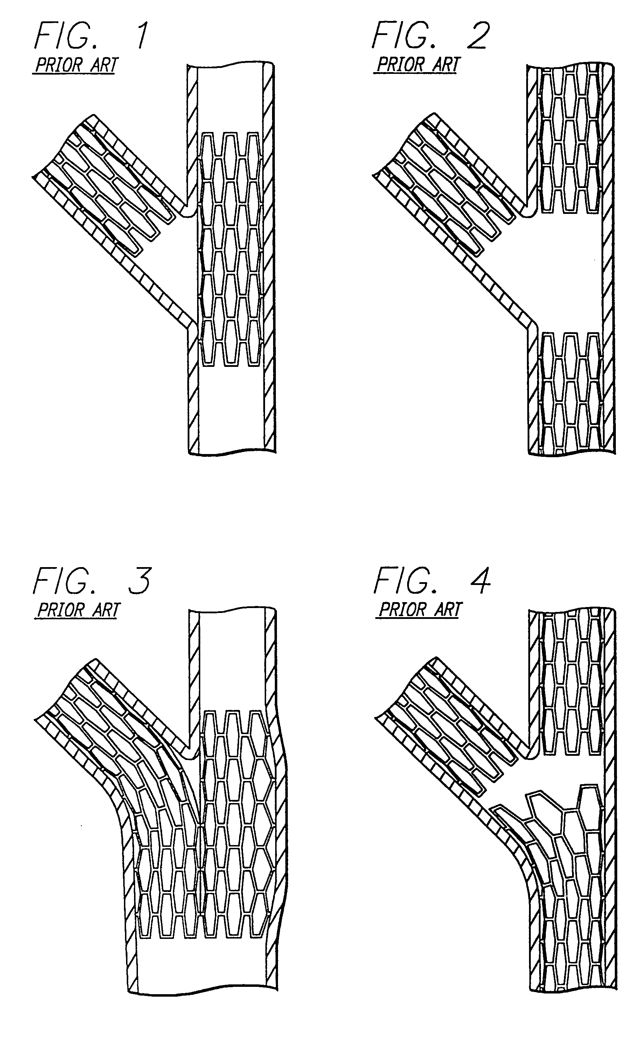

The present invention is directed to an assembly for treating bifurcations in, for example, the coronary arteries, veins, arteries, and other vessels in the body. Prior art attempts at implanting intravascular stents in a bifurcation have proved less than satisfactory. For example, FIGS. 1-4 depict prior art devices which include multiple stents being implanted in both the main vessel and a side-branch vessel. In FIG. 1, a prior art "T" stent is implanted such that a first stent is implanted in the side branch near the ostium of the bifurcation, and a second stent is implanted in the main vessel, across the side-branch ostium. With this approach, portions of the side-branch vessel are left uncovered, and blood flow to the side-branch vessel must necessarily pass through the main-vessel stent, causing possible obstructions or thrombosis.

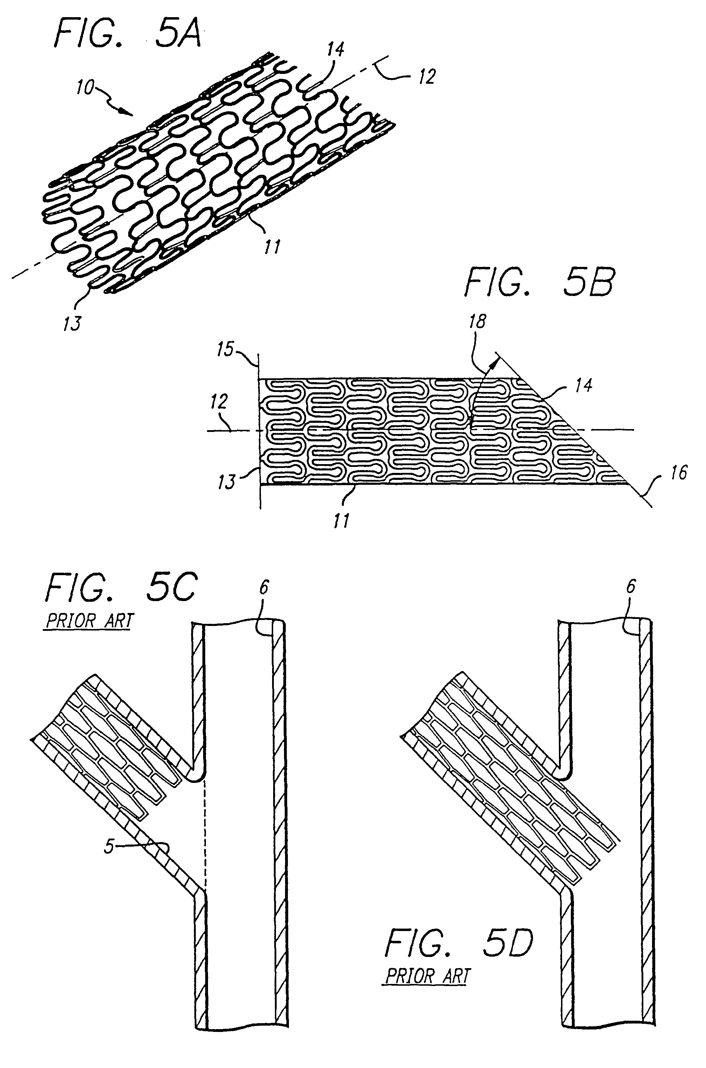

Referring to FIG. 2, three prior art stents are required to stent the bifurcation. In FIG. 3, the prior art method includes implanting two stents side by side, such that one stent extends into the side-branch vessel and the main vessel, and the second stent is implanted in the main vessel. This results in a double-barreled lumen which can present problems such as thrombosis, and turbulence in blood flow. Referring to the FIG. 4 prior art device, a first stent is implanted in the side-branch vessel, a second stent is implanted in a proximal portion of the main vessel, and a third stent is implanted distal to the bifurcation, thereby leaving a small gap between the stents and an uncovered luminal area.

All of the prior art devices depicted in FIGS. 1-4 have various drawbacks.

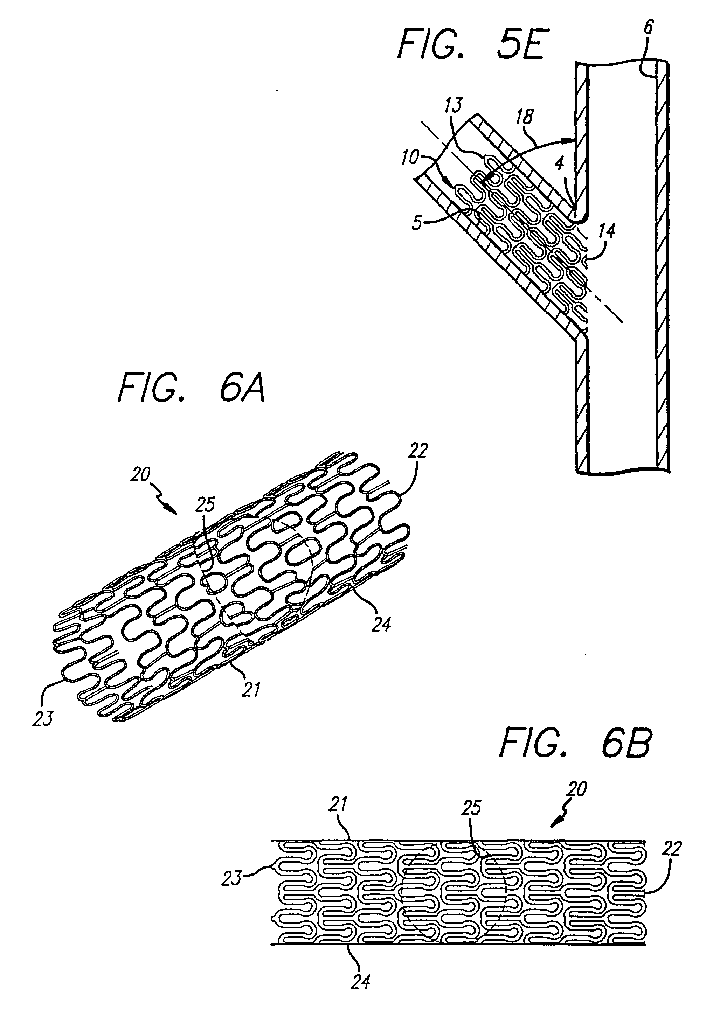

As depicted in FIGS. 5A, 5B and 5E, a proximal-angled stent 10 is configured for deployment in a side-branch vessel 5. The proximal-angled stent 10 includes a cylindrical member 11 having a longitudinal axis 12 which is an imaginary axis extending through the cylindrical member 11. A distal end 13 and a proximal end 14 define the length of the cylindrical member 11. A first plane section 15 is defined by a plane section through the distal end 13 of the cylindrical member, and second plane section 16 is defined by a plane section through the proximal end 14 of the cylindrical member. A second plane section 16 defines an acute angle 18, which is the angle between the second plane section 16 and the longitudinal axis 12.

In treating the side-branch vessel 5, if a prior art stent is used in which there is no acute angle at one end of the stent to match the angle of the bifurcation, a condition as depicted in FIGS. 5C and 5D will occur. That is, a stent deployed in the side-branch vessel 5 will leave a portion of the side-branch vessel exposed (FIG. 5C), or as depicted in 5D, a portion of the stent will extend into the main-vessel 6. As depicted in FIG. 5E, the proximal-angled stent 10 has an acute angle 18 that approximates the angle formed by the bifurcation 4 of the side-branch vessel 5 and the main-vessel 6. Thus, the acute angle 18 is intended to approximate the angle formed by the intersection of the side-branch 5 and the main-vessel 6. The angle between the side-branch vessel 5 and the main-vessel 6 will vary for each application, and for the present purposes, should be less than ninety degrees (90°). If there is a ninety degree (90°) angle between the side-branch vessel and the main vessel, a conventional stent having ends that are transverse to the stent longitudinal axis, would be suitable for stenting the side-branch vessel.

The proximal-angled stent can be implanted in the side-branch vessel to treat a number of angulated ostial lesions including, but not limited to, the following:
1. The ostium of a left anterior descending artery (LAD) where there is a circumflex or trifurcation vessel at less than ninety degrees (90°) in its departure from the LAD.
2. The ostium of the circumflex artery or a trifurcation in a similar situation as number 1.
3. The ostium of a sizeable diagonal.
4. The LAD just distal to, but sparing, the origin of a diagonal.
5. The ostium of a circumflex marginal artery with an angulated take-off.
6. Disease in the circumflex artery just distal to a marginal take-off, but sparing that take-off.
7. The aorta-ostium of a right coronary artery with an angled take-off.
8. The origin of an angulated posterior descending artery.
9. The origin of an LV extension branch just at and beyond the crux, sparing the posterior descending artery.
10. The ostium of an angulated vein graft origin.
11. Any of many of the above locations in conjunction with involvement of the bifurcation and an alternate vessel.

The proximal-angled stent typically can be used as a solo device to treat the foregoing indications, or it can be used in conjunction with the main-vessel stent described herein for stenting the bifurcation.

As depicted in FIGS. 6A and 6B, the main-vessel stent 20 is configured for deployment in the main vessel 6. The main-vessel stent 20 includes a cylindrical member 21 having a distal end 22 and a proximal end 23. The main-vessel stent 20 includes an outer wall surface 24 which extends between a distal end 22 and a proximal end 23 and incorporates an aperture 25 on the outer wall surface 24. The aperture 25 is configured so that, upon expansion, it approximates the diameter of the expanded proximal end 14 of the proximal-angled stent 10. When the main-vessel stent 20 is implanted and expanded into contact with the main-vessel 6, the aperture 25 is aligned with the side-branch vessel 5 and the proximal end 14 of the proximal-angled stent 10, thereby providing an unrestricted blood flow path from the side-branch vessel to the main vessel. Unlike what has been available in the prior art, the main-vessel catheter allows selection and positioning of an aperture at the side-branch ostium. Further, it provides for the positioning ofa guide wire during main-vessel stent deployment which can be used for additional intervention if necessary. In the prior art techniques, access to a side-branch is through a randomly selected stent element ("cell"), and is only possible after deployment of the stent. The precise positioning of aperture 25 in the stent illustrated in FIGS. 6A and 6B is optional and the aperture 25 could be positioned either closer to the proximal end or closer to the distal end of the main vessel stent 20.

The proximal-angled stent 10 and the main-vessel stent 20 can be formed from any of a number of materials including, but not limited to, stainless steel alloys, nickel-titanium (NiTi) alloys (the NiTi can be either a shape memory type or pseudoelastic), tantalum, tungsten, or any number of polymer materials. Such materials of manufacture are known in the art. Further, the proximal-angled stent 10 and the main-vessel stent 20 can have virtually any pattern known to prior art stents. In a preferred configuration, the proximal-angled stent 10 and the main-vessel stent 20 are formed from a stainless steel material and have a plurality of cylindrical elements connected by connecting members, wherein the cylindrical elements have an undulating or serpentine pattern. Such a stent is disclosed in U.S. Patent No. 5,514,154 and is manufactured and sold by Advanced Cardiovascular Systems, Inc., Santa Clara, California. The stent is sold under the tradename MultiLink® Stent. Such stents can be modified to include the features of the proximal-angled stent 10 (the angulation) and the main-vessel stent 20 (the aperture).

The proximal-angled stent 10 and the main-vessel stent 20 preferably are balloon-expandable devices that are mounted on a balloon portion of a catheter and crimped tightly onto the balloon to provide a low profile delivery diameter. After the catheter is positioned so that the stent and the balloon portion of the catheter are located either in the side-branch or the main vessel, the balloon is expanded, thereby expanding the stent beyond its elastic limit into contact with the vessel. Thereafter, the balloon is deflated and the balloon and catheter are withdrawn from the vessel, leaving the stent implanted. Deployment of the angled and main-vessel stents is accomplished by a stent delivery system adapted specifically for treating bifurcated vessels. The proximal-angled stent and the main-vessel stent could be made to be either balloon expandable or self-expanding.

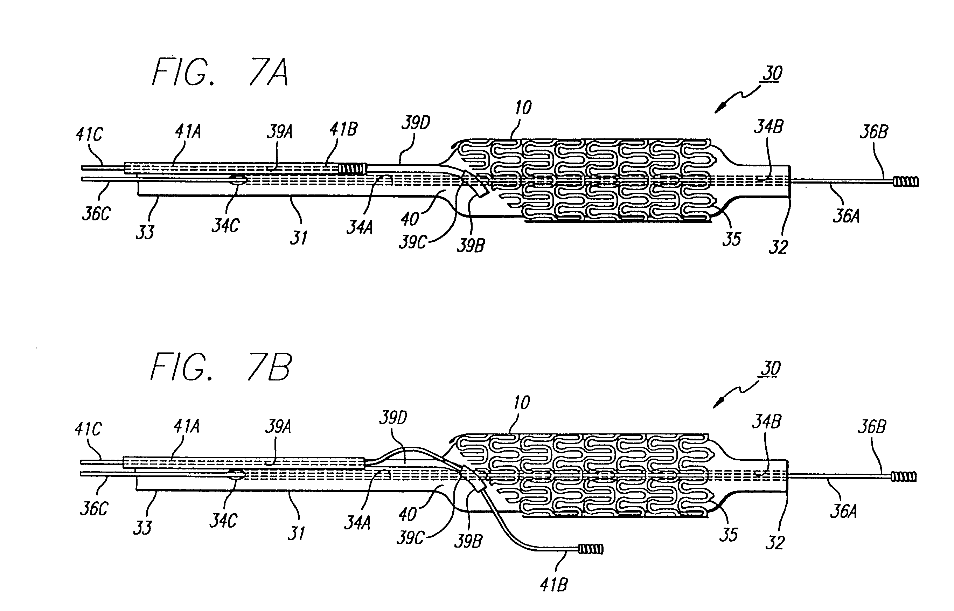

In one preferred arrangement for delivering the stents, as depicted in FIGS. 7A and 7B, the side-branch stent delivery assembly 30 is provided and includes side-branch catheter 31. The side-branch catheter includes a distal end 32 which is configured for delivery into the patient's vasculature and a proximal end 33 which remains outside the patient. A first guide wire lumen 34A extends through at least a portion of the side-branch catheter 31, depending on the type of catheter (e.g., rapid exchange, over-the-wire, etc.) desired for a particular application. The first guide wire lumen 34A preferably is defined by a distal end 34B and a side port 34C, which is typical of the so-called rapid-exchange-type catheters. Typically, a slit (not shown) extends from the side port 34C to just proximal of the balloon portion of the catheter, so that the catheter can be rapidly exchanged during a medical procedure, as is known.

The expandable member 35, which typically is a non-distensible balloon, has a first compressed diameter for delivery through the vascular system, and a second expanded diameter for implanting a stent. The expandable member 35 is positioned near the distal end 32, and in any event between the distal end 32 of the first catheter 31 and the side port 34C.

Referring to FIGS. 7A and 7B, a tracking guide wire 36A having a distal end 36B and a proximal end 36C extends through the first guide wire lumen 34A. The tracking guide wire 36A preferably is a stiff wire having a diameter of 0.356 mm (.014 inch), but can have a different diameter and stiffness as required for a particular application. An especially suitable guide wire can be one of those manufactured and sold under the tradenames Sport® and Ironman®, manufactured by Advanced Cardiovascular Systems, Inc., Santa Clara, California. The tracking guide wire 36A is sized for slidable movement within the first guide wire lumen 34A.

The side-branch delivery assembly 30 further includes a second guide wire lumen 39A, which is associated with the expandable member 35. The second guide wire lumen 39A includes an angle portion 39B and a straight portion 39C, and is firmly attached to an outer surface 40 of the catheter 31, at a point just proximal to the expandable member 35. An integrated stent-positioning guide wire 41A is sized for slidable movement within the second guide wire lumen 39A. A slit 39D is formed in the lumen 39A near its distal end, so that the stiff guide wire 41A can bow outwardly as shown in FIG. 7B. The portion of the guide wire 41A that bows out of the slit 39D will limit the advancement of the catheter 31, as will be further described infra. The integrated stent-positioning guide wire 41 A has a distal end 41B, and a proximal end 41C which extends out of the patient. Again, it is preferred that the integrated stent-positioning guide wire, 41A be a fairly stiff wire as previously described, for the reasons set forth below in delivering and implanting the stents in the bifurcation.

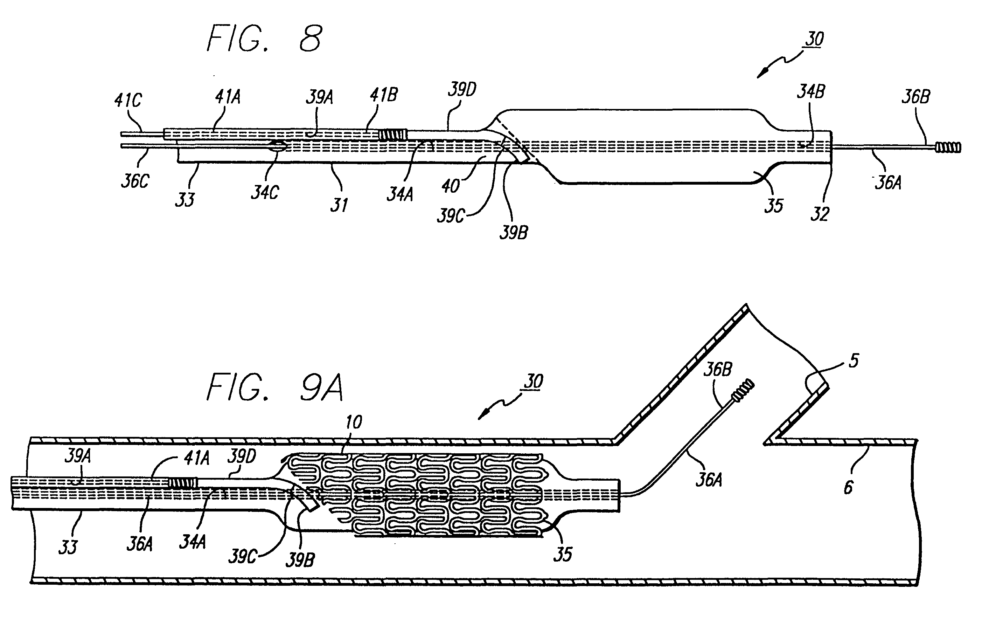

In an alternative arrangement, the catheter 31 can have an angled expandable member 42 as depicted in FIG. 8. The proximal end of the expandable member is angled to coincide with the angle of the proximal-angled stent 10 (not shown in FIG. 8 for clarity). This arrangement is particularly useful in delivering the angled stent since the second guide wire lumen 39A, and its angled portion 39B, have the same angle as the stent and the proximal end of the expandable member.

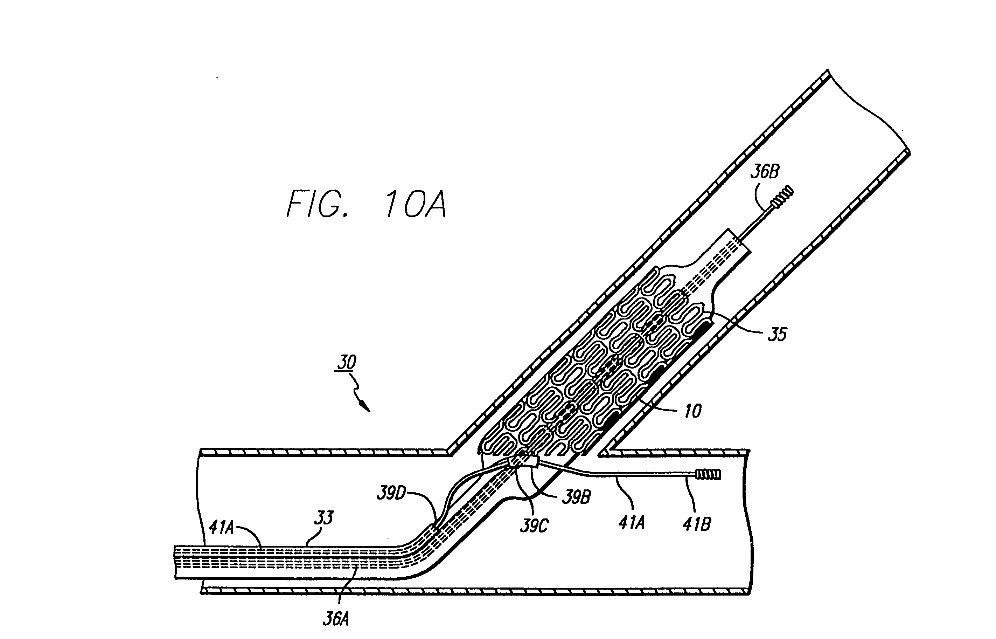

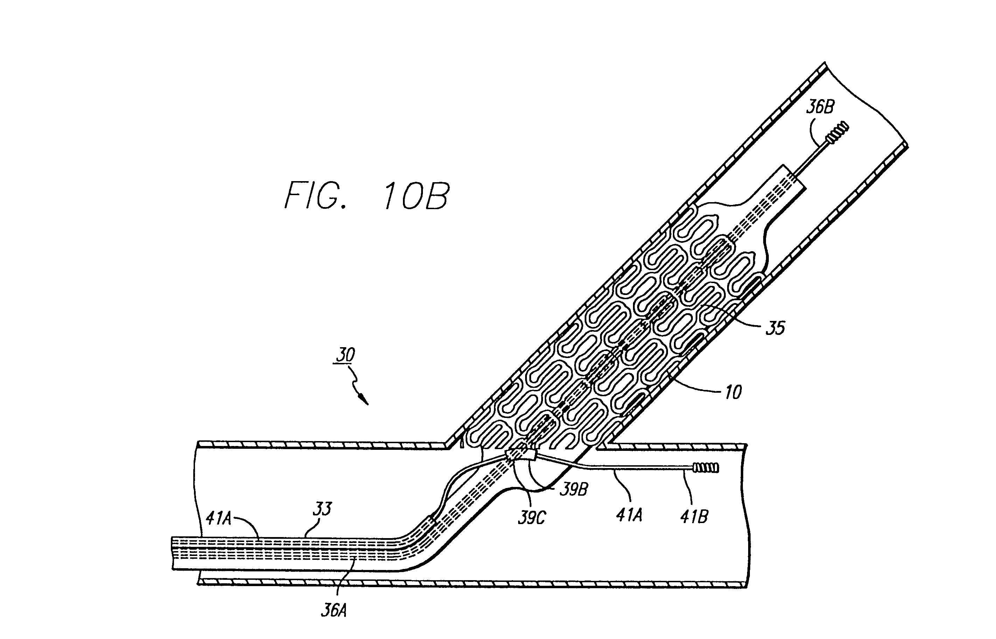

As depicted in FIGS. 9A-11D, the proximal-angled stent 10 is mounted on the side-branch catheter 31 and implanted in the side-branch vessel 5. The method of achieving proximal-angled stent implantation is as follows.

The proximal-angled stent 10 first is crimped tightly onto the expandable member 35 for low-profile delivery through the vascular system.

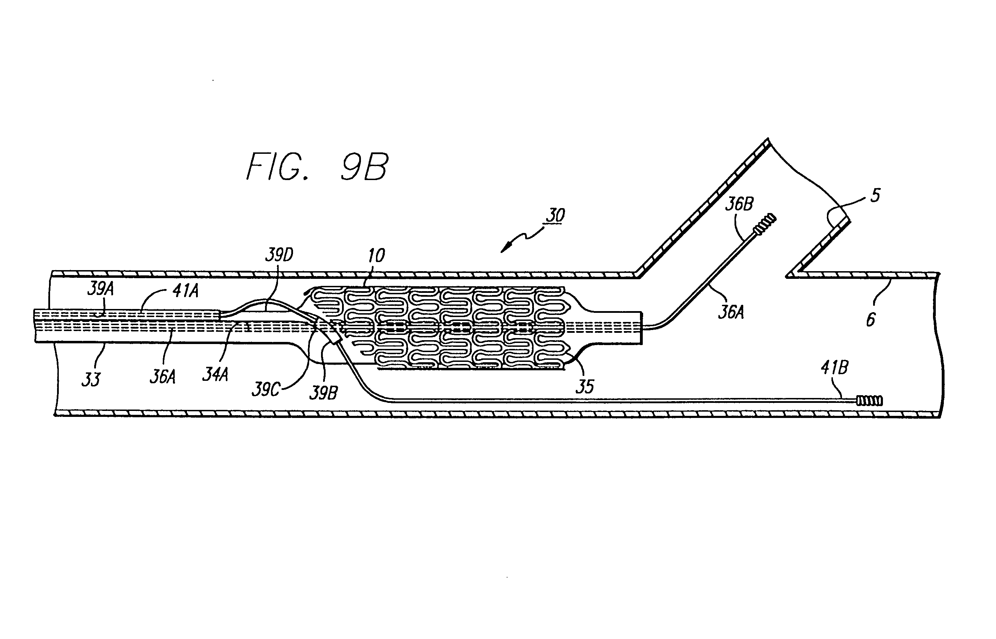

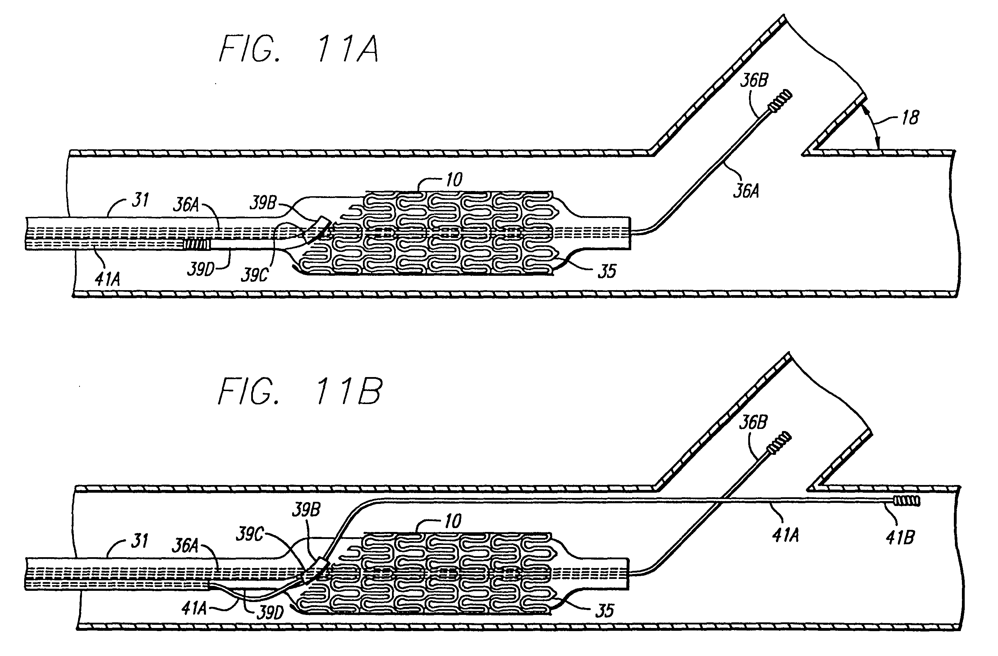

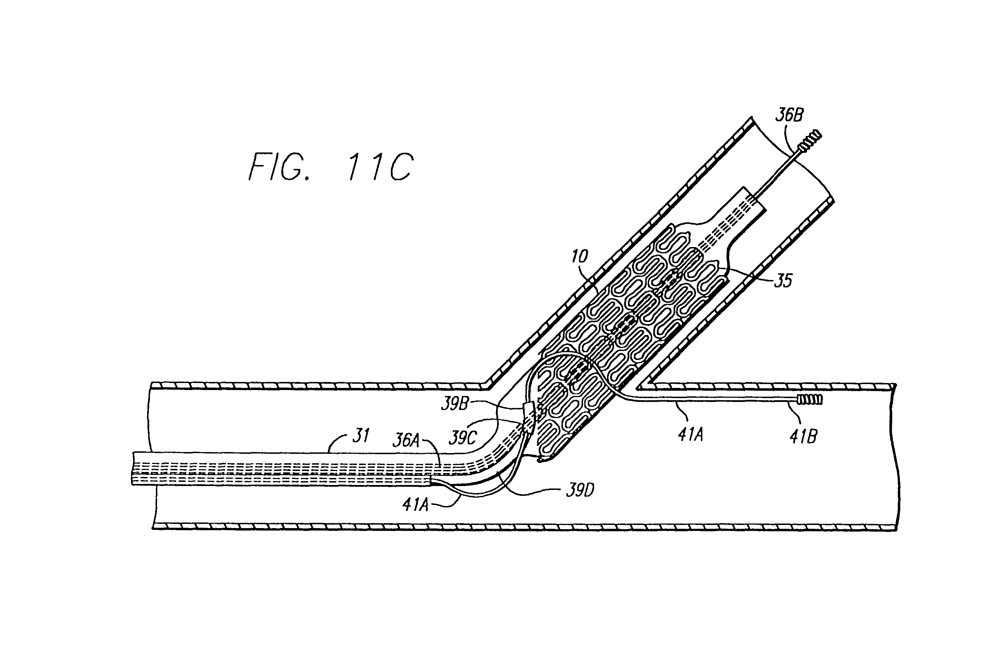

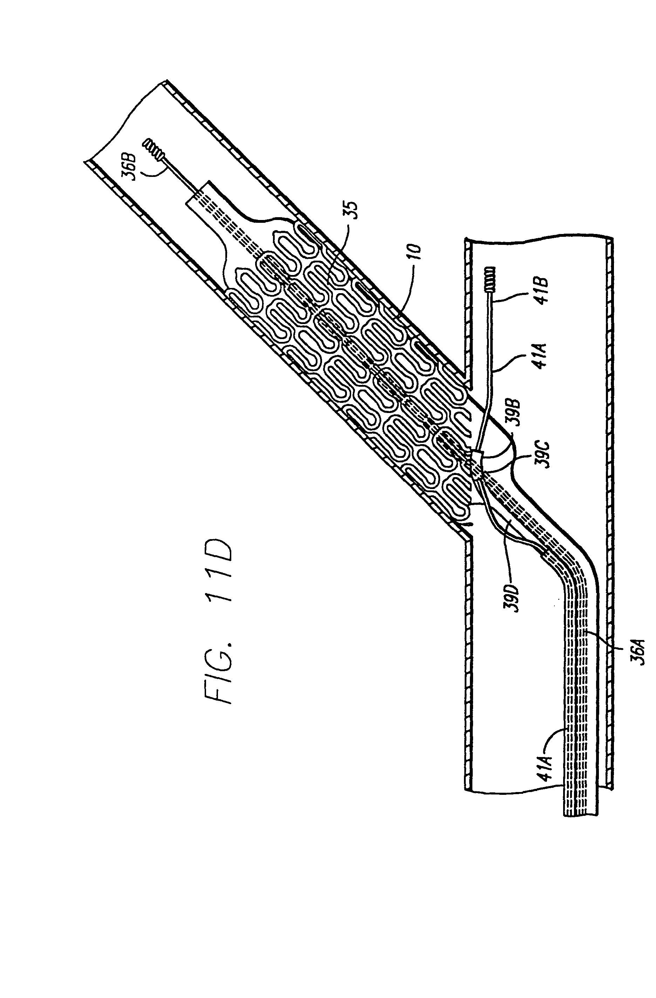

The distal end 36B of the guide wire 36A is advanced into the side-branch vessel 5 and distally of the target area, with the proximal end 36C remaining outside the patient. The side-branch catheter 31 then is advanced within a guiding catheter (not shown) along the tracking guide wire 36A until the distal end 32 of the catheter is just proximal (about 1 cm) of entering the side-branch vessel 5. Up to this point, the guide wire 41A resides in the second guide wire lumen 39A so that the distal end 41B of the wire preferably is near, but is not in, the angled portion 39B of the guide wire lumen 39A. This method of delivery prevents the two guide wires from wrapping around each other, the guide wire 41 A being protected by the catheter during delivery. The distal end 41B of the integrated stent positioning guide wire 41A then is advanced by having the physician push the proximal end 41 C from outside the patient's body. The distal end 41B of the integrated stent-positioning guide wire travels through the guide wire lumen 39A and the angled portion 39B and passes close to the proximal end 14 of the angled stent 10 and the expandable member 35 and exits at the angled portion 39B of the second guide wire lumen 39B. As the guide wire 41A is advanced into, through and out of the angled portion 39B, the stiffness of the wire causes it to bow outwardly through the slit 39D in the distal portion of the second guide wire lumen 39A. Thus, as can be seen, for example, in FIGS. 9B, 10A, 10B, and 11B-11D, the positioning guide wire 41 A bows outwardly and, due to its stiffness, provides a bumper against the ostium of the side-branch vessel to assist in positioning and deploying the stents. The stent-positioning guide wire 41A is advanced in the main vessel until the distal end 41B is distal to the side-branch vessel 5. The catheter then is advanced into the side-branch vessel 5 until resistance is felt, caused by the stent-positioning guide wire 41 A pushing up against the ostium of the side-branch vessel 5. As previously described, the stent-positioning wire 41A is relatively stiff, as is the tracking guide wire 36A, so that each can properly orient the side-branch catheter 31 as it is advanced into the side-branch vessel 5. The angled portion 39B of the second guide wire lumen 39A is angled to assist in rotating the side-branch catheter 31 into proper position into the side-branch vessel 5. If the stent approaches the side-branch vessel 5 in the incorrect position, as depicted in FIGS. 11A-11B, the stent-positioning wire 41A would be forced to make a very acute angle. The wire stiffness, however, prevents this from happening and causes the wire to assume the position of least stress. As is illustrated in FIGS. 11C-11D, to relieve this stress buildup, the wire 41A creates a torque on the angled portion 39B, causing the guide wire lumen 39A and the side-branch catheter 31, with the proximal-angled stent 10, to rotate into the correct position. Preferably, the slit 39D is formed on the outer surface 40 of the catheter 31 near the angled portion 39B, so that the stent-positioning guide wire 41A can bow outwardly out of the slit 39D, thereby increasing the ability to torque the catheter and the proximal-angled stent.

Thereafter, the proximal-angled stent 10 mounted on the expandable member 35 is aligned across the target area, and viewed under fluoroscopy, the acute angle 18 on the proximal end of the proximal-angled stent is aligned at the intersection of the side-branch vessel 5 and the main-vessel 6 (the ostium of the side-branch vessel) so that the proximal-angled stent completely covers the target area in the side-branch vessel 5, yet does not extend into the main-vessel 6, thereby compromising blood flow. The expandable member 35, which typically is a non-distensible balloon, is expanded by known methods, thereby expanding the proximal-angled stent into contact with the side-branch vessel 5, and thereby implanting the proximal-angled stent in the side-branch vessel. Thereafter, the expandable member 35 is deflated and the side-branch catheter assembly 31 is withdrawn from the patient's vasculature. The side-branch catheter 31 is designed so that both the tracking guide wire 36A and the stent-positioning guide wire 41A can be left in the vessels in which each respectively has been deployed, in the event sequential or simultaneous high pressure balloon inflation be required in each of the vessels in order to complete the stenting procedure. In other words, the integrated positioning wire can be unzipped through the slit (not shown) from the proximal 100 cm of the catheter, thereby allowing it to act as a rapid exchange wire. Preferably, high pressure balloons are inflated simultaneously in the main-vessel stent and the proximal-angled stent, in order to avoid deforming one stent by unopposed balloon inflation occurring in the other one. This additional step is a matter of physician preference. Using this method, the side-branch vessel 5 can be stented without the need for stenting the main vessel, as shown in FIGS. 11A-11D.

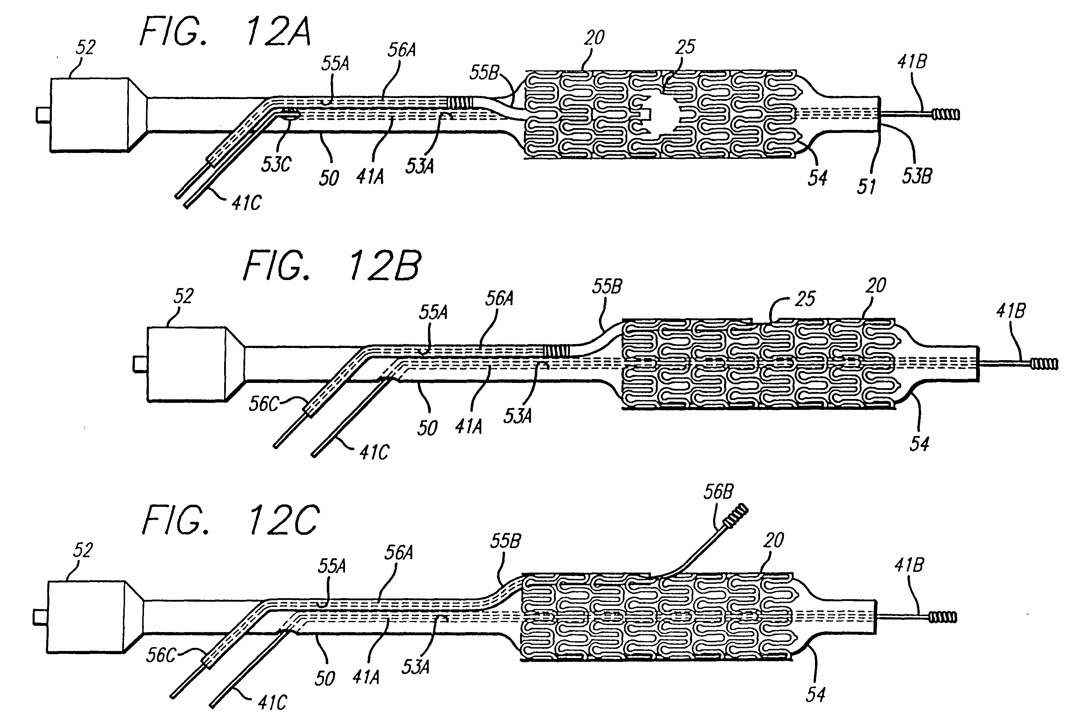

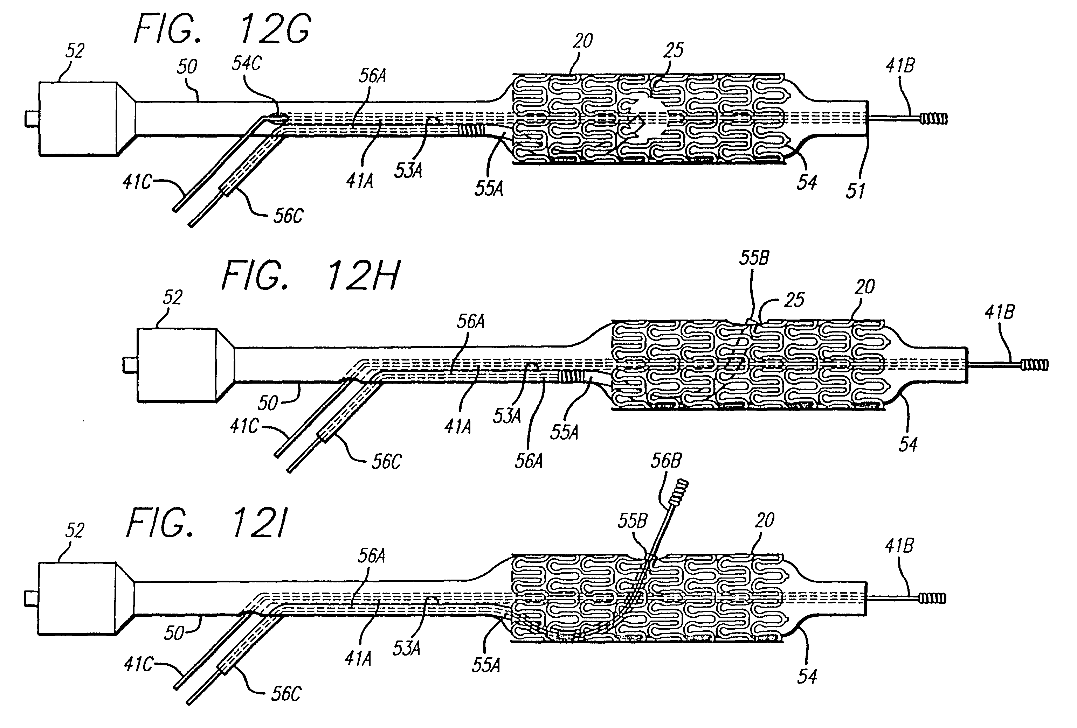

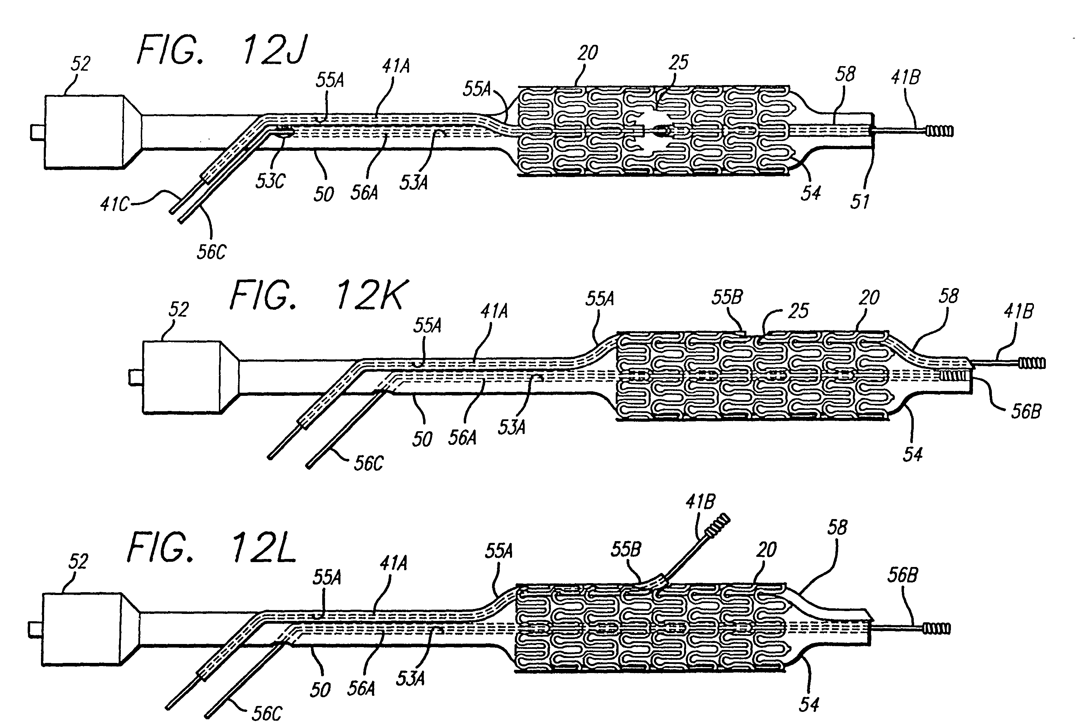

If necessary, the main-vessel 6 also can be stented after stenting the side-branch vessel. In that regard, a main-vessel catheter assembly 50 is provided for implanting the main-vessel stent 20, as depicted in FIGS. 12A to 13E. In one preferred arrangement, as shown in FIGS. 12A-12C, the main-vessel catheter 50 includes a distal end 51 which is configured for advancement within the patient's vasculature, and a proximal end 52 which remains outside the patient. The main-vessel catheter includes a guide wire lumen 53A having a distal end 53B and a side port 53C, which is proximal to the balloon portion of the catheter. The side port 53C is provided in a so-called rapid-exchange catheter system which includes a slit (not shown) as is known in the art. An expandable member 54 is located near the distal end 51 of the main-vessel catheter 50. Typically, the expandable member 54 is a non-distensible balloon of the type known in the art for delivering and expanding stents.

The positioning guide wire lumen 55A is positioned partly on the catheter shaft and partly on the expandable member 54, and is configured for slidably receiving the integrated stent-positioning guide wire 56A. Prior to stent delivery, the integrated stent-positioning guide wire 56A resides in the positioning guide wire lumen 55A and only during stent delivery is it then advanced into and through the angled portion 55B of the lumen.

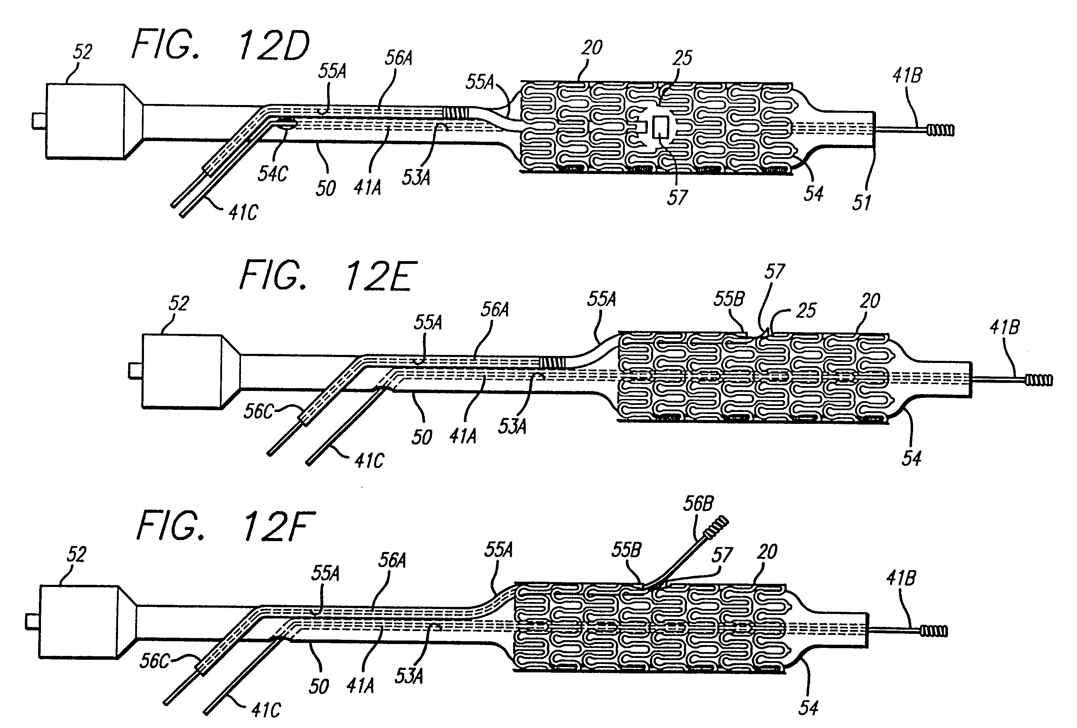

Other preferred arrangements, for implanting the main-vessel stent 20 in the main vessel 6 are depicted, for example, in FIGS. 12D-12F. This arrangement is identical to that depicted in FIGS. 12A-12C, with the addition of a ramp 57 which is mounted on the balloon 54 and which provides a slight incline for the integrated stent-positioning guide wire 56A as it exits the positioning guide wire lumen 55A. As the guide wire 56A slides along the ramp 57, the distal portion 56B of the guide wire will move radially outwardly which helps position the guide wire and orient it into the side-branch vessel. In another arrangement for implanting the main-vessel stent in the main vessel, as depicted in FIGS. 12G-12I, the guide wire lumen 55A passes underneath the main-vessel stent 20 and on top of the balloon 54. The distal end angled portion 55B curves along the balloon so that, as the distal end of the guide wire 56B advances out of the distal end angled portion 55B of the lumen, the distal end of the guide wire is traveling radially outwardly so that it can more easily locate and advance into the side-branch vessel 5.

In still another arrangement for implanting the main-vessel stent 20 in the main-vessel 6, as depicted in FIGS. 12J-12L, the positioning guide wire lumen 55A is positioned under the stent 20 and terminates at the distal end angled portion 55B in the middle of the aperture 25. The distal end angled portion 55B of the guide wire lumen will spring outwardly, facilitating the advancement of the distal portion ofthe integrated stent positioning guide wire 56B into the side branch vessel. A distal guide wire lumen 58 is attached to the outer surface of balloon 54 and extends from the aperture 25 to essentially the distalmost end of the catheter.

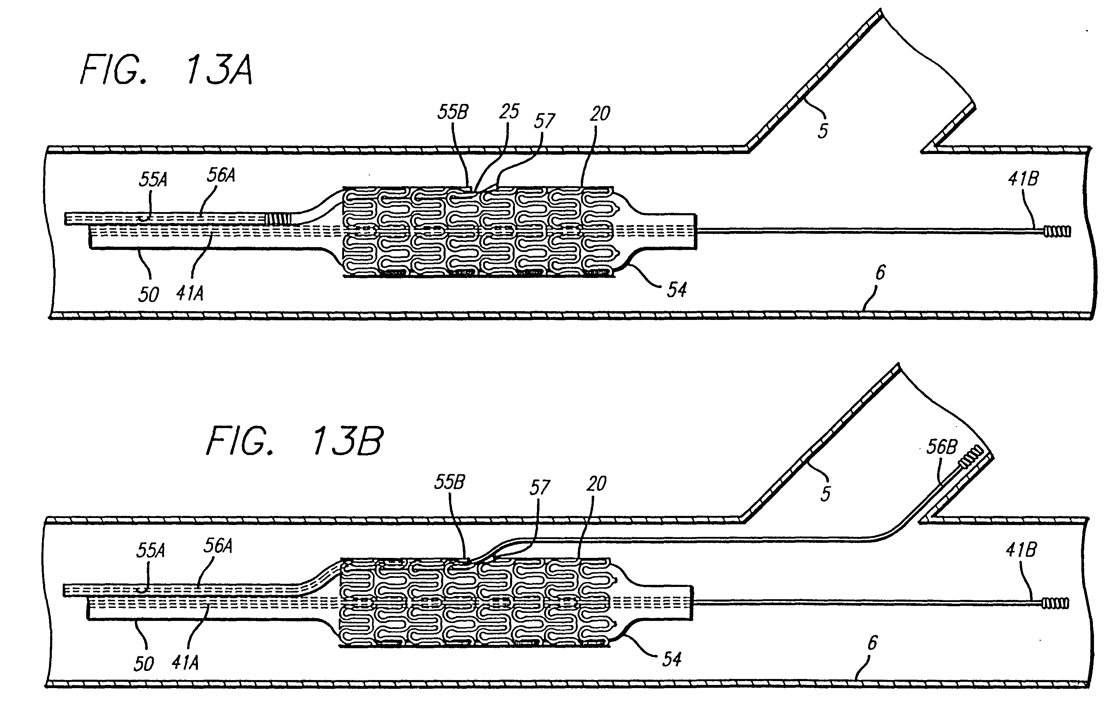

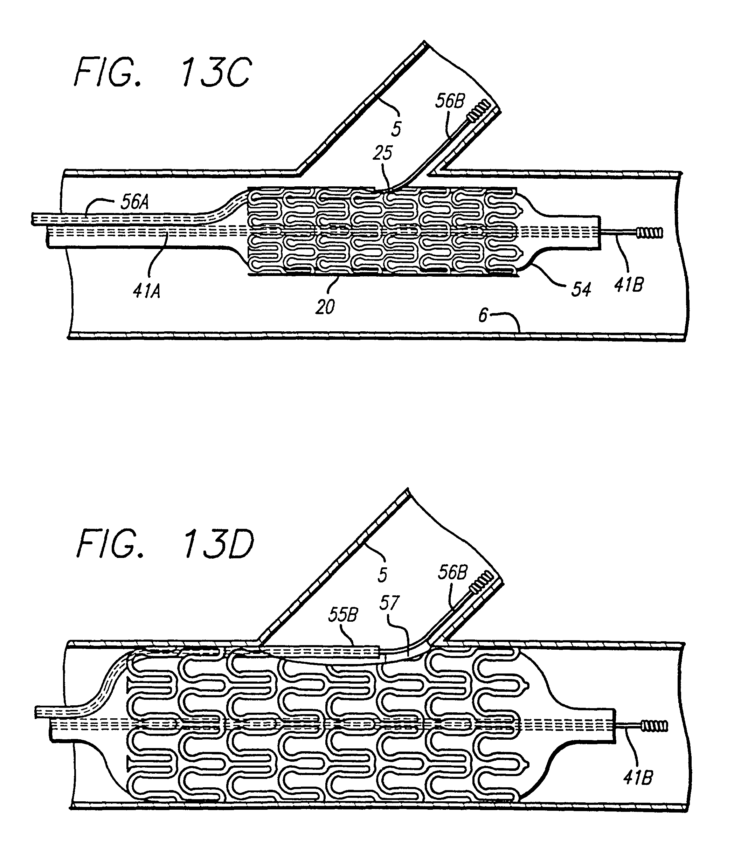

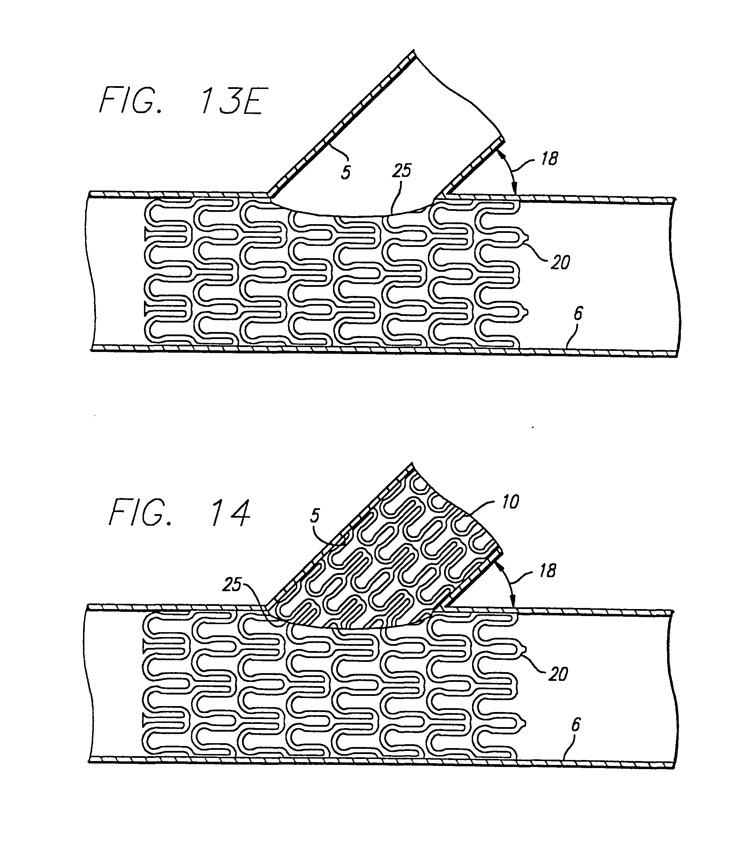

In one preferred arrangement for implanting the main-vessel stent 20 in the main-vessel 6, as depicted in FIGS. 12A-12I and 13A-13D, the integrated stent-positioning guide wire 41 A of the side-branch delivery assembly 41 A remains in position in the main-vessel 6, while the side-branch tracking guide wire 36A is withdrawn from the patient. The main-vessel catheter 50 is back loaded onto the guide wire 41A by inserting the proximal end 41C of the wire into the distal end of the catheter and into the guide wire lumen 53A. The main-vessel catheter 50 is advanced over the guide wire 41A and viewed under fluoroscopy until the main-vessel stent 20 is positioned in the main-vessel 6, just proximally of the side-branch vessel 5. The distal portion 56B of the integrated stent-positioning guide wire 56A of the main-vessel catheter then is advanced by the physician who pushes on the proximal portion 56C from outside the patient's body. The distal portion 56B of the guide wire 56A advances into and through the positioning guide wire lumen 55A, and passes underneath the proximal end of the main-vessel stent 20 and exits the angled portion 55B of the positioning guide wire of the lumen 56A and enters the side-branch vessel 5. The main-vessel catheter 50 then is advanced distally into the main vessel until resistance is felt, caused by the integrated stent-positioning guide wire of the main-vessel catheter 56A pushing up against the ostium of the side-branch vessel. The stiffness of the stent-positioning guide wire 56A causes the main-vessel catheter 50, with the main-vessel stent 20 thereon, to rotate, so that the aperture 25 faces the ostium of the side-branch vessel 5 where the proximal-angled stent 10 already is implanted.

The expandable member 54, which typically is a non-distensible expandable balloon, is inflated, thereby expanding the main-vessel stent 20 into contact with the main-vessel 6. The aperture 25 correspondingly expands and, when properly aligned, provides a blood flow path between the aperture 25 and the proximal-angled stent 10 implanted in the side-branch vessel 5. As can be seen in FIGS. 12A-12I and 13A-13D, the positioning guide wire lumen 55A is positioned on the expandable member 54, such that when the expandable member is inflated, the positioning guide wire lumen 55A does not interfere with the implanting of the main-vessel stent 20. After the main-vessel stent is implanted in the main vessel, the expandable member 54 is deflated, and the main-vessel catheter 50 is withdrawn from the patient. As seen in FIG. 14, the bifurcated vessel has been fully covered by the stents, the side-branch vessel 5 being covered by the proximal-angled stent 10, and the main-vessel 6 being covered by the main-vessel stent 20, so that no portion of the bifurcation 4 is left uncovered and there is no overlap in the implanted stents.

In an alternative method of implanting the main-vessel stent 20 in the main-vessel 6 as depicted in FIGS. 12J-12L, the integrated stent-positioning guide wire 41A of the side-branch delivery assembly, which here serves as the tracking guide wire, is advanced through the positioning guide wire lumen 55A and the distal guide wire lumen 58 attached to the balloon so that the guide wire advances distally of the distal end 51 of the main-vessel catheter. Thus, the guide wire distal end 41B is advanced into the main vessel so that it is distal ofthe side-branch vessel 5. The integrated stent-positioning guide wire 56A, which until this point has remained within the guide wire lumen 53A (see FIG. 12K), is advanced distally as depicted in FIG. 12L and is advanced into the main vessel distally of the side-branch vessel. The guide wire 41A then is withdrawn proximally through the guide wire lumen 58 until guide wire distal end 41B is able to exit the guide wire lumen distal end angled portion 55B, as shown in FIG. 12L. Because the angled portion of the positioning guide wire lumen 55B is preformed and has bias, it will spring outwardly. The guide wire 41A then can be advanced into the side-branch vessel for further positioning. As the catheter 50 is advanced over the guide wires, the distal portion 41B of the guide wire will push against the ostium of the side-branch vessel thereby insuring the location of the main-vessel stent 20 and, importantly, the aperture 25 will align with the opening to the side-branch vessel 5.

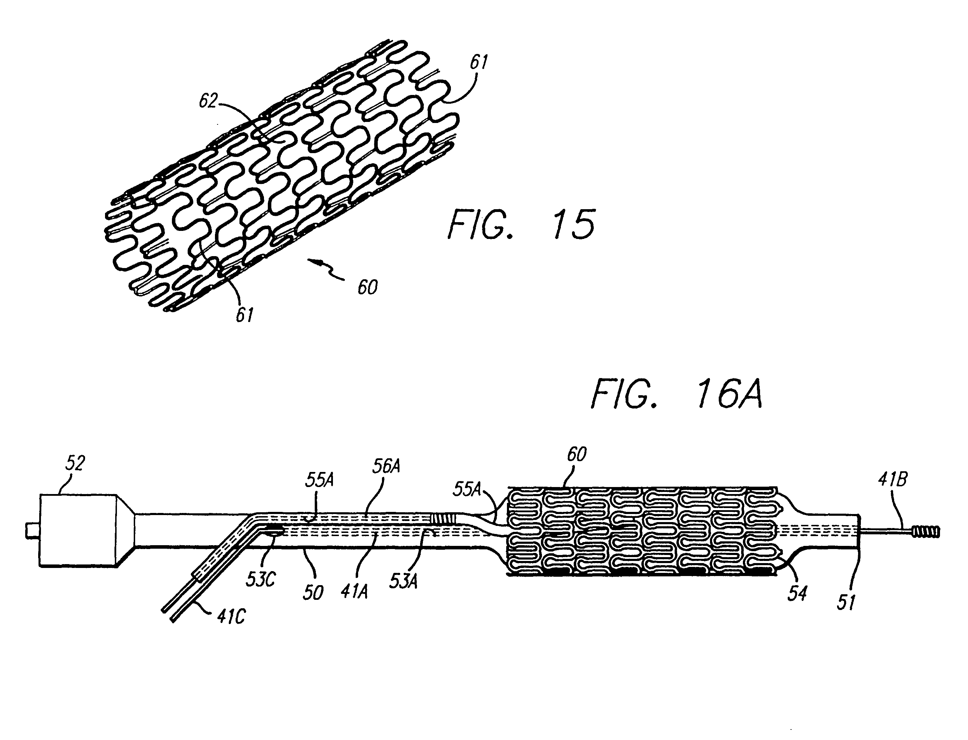

A non-angulated stent (see FIG. 15) can be implanted using the catheter system of FIGS. 7A-11D for stenting a side-branch vessel having an origin approaching ninety degrees (90°) in its takeoff from the main vessel. In this circumstance the positioning wire serves solely to arrest the forward movement of the stent precisely at the origin of the vessel for more precise positioning. However, the acute angle 18 is appropriate for a bifurcated vessel 4 in which the angulation is the acute angle 18, or less than ninety degrees (90°). Thus, consideration could be given to standard thirty degree (30°), forty-five degree (45°), and sixty degree (60°) angled stent designs for the proximal-angled stent 10, which should provide sufficient luminal wall coverage. The proximal-angled stent 10 has a wide range of applicability and can be used for stenting ostial side-branch lesions, ostial circumflex or left anterior descending (LAD) lesions where the bifurcation is an acute angle, or less than ninety degrees (90°), and ostial lesions involving the angulated origin of a right coronary or vein graft. Importantly, the stents provide full coverage of the ostial intima without protruding into the main vessel and without compromising subsequent access to the distal portion of the main vessel.

In order to assist in properly aligning both the proximal-angled stent 10 and the main-vessel stent 20 in the side-branch vessel 5 and the main-vessel 6, respectively, the positioning guide wire lumen 39A, on the side-branch catheter 31, and the positioning guide wire lumen 55A, on the main-vessel catheter 50, can be radiopaque, or can have a radiopaque marker associated therewith so that each is visible under fluoroscopy. Thus, when advancing the side-branch catheter 31 and the main-vessel catheter 50, the proper orientation can be determined more easily by viewing the position of the positioning guide wire lumen 39A in connection with the main-vessel 6 or the positioning guide wire lumen 55A in connection with aligning the aperture 25 with the side-branch vessel 5. Additionally, the positioning guide wire 56A for positioning the main-vessel stent 20 and the positioning guide wire 41A for positioning the angled stent 10 either are radiopaque or have radiopaque portions, such as gold markers, to assist in positioning and orienting the catheters and stents during deployment and implantation.

While the foregoing description includes implanting the proximal-angled stent 10 in the side-branch vessel 5 prior to implanting the main-vessel stent 20 in the main-vessel 6, in an alternative arrangement, the order of the implanting procedures can be reversed. However, it should be understood that by implanting the main-vessel stent 20 in the main-vessel 6, and subsequently by implanting the proximal-angled stent 10 in the side-branch vessel 5, the aperture 25 must he carefully aligned with the side-branch vessel 5 so that the side-branch catheter 31 can be advanced through the expanded main-vessel stent 20 and the aperture 25 and into the side-branch vessel 5 for implanting the proximal-angled stent 10.

While the side-branch catheter 31 and the main-vessel catheter 50 have been described herein as being of the rapid-exchange type, they also can be of a conventional over-the-wire-type catheter. In over-the-wire-type catheters, the guide wire lumen extends from the distal end of the catheter to the proximal end with no side port as is found in the rapid-exchange-type catheters. Typical of over-the-wire-type catheters is the type disclosed in U.S. Patent Nos. 4,323,071 and B1 4,323,071, commonly assigned and commonly owned by Advanced Cardiovascular Systems, Inc., Santa Clara, California.

In one preferred arrangement, as depicted in FIG. 15, a main-vessel unmodified stent 60 can be configured without the side aperture 25 of the main-vessel stent 20. Upon expansion, the individual strut members 61 of unmodified stent 60 expand sufficiently to permit a balloon catheter to be inserted therethrough, and expanded, to form an aperture which corresponds to the opening to the side-branch vessel 5.

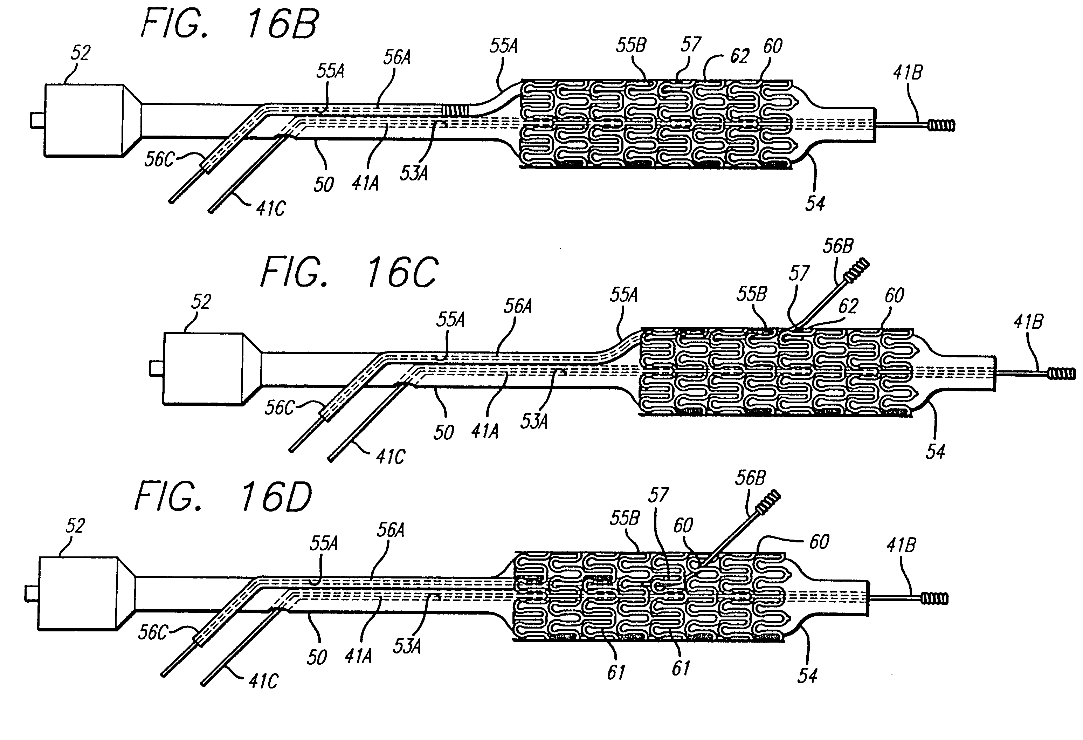

In one method of stenting the bifurcation, the side-branch vessel 5 is first stented as described, for example, in the manner shown in FIGS. 9A through 11D. Thereafter, the main-vessel 6 is stented with the unmodified main-vessel stent 60, which does not have an aperture formed in the side of the stent. As shown in FIGS. 15-18, the unmodified stent 60 is mounted on the expandable portion 54 of the main-vessel catheter 50. The main-vessel catheter 50 is back loaded onto the proximal end of the integrated stent-positioning guide wire of the side-branch delivery assembly 41A which already is in position in the main vessel. The main-vessel catheter 50 is advanced over the guide wire and is viewed under fluoroscopy until the stent 60 is positioned in the main vessel about 1 cm proximally of the side-branch vessel. The distal end 56B of the integrated stent-positioning guide wire 56A then is advanced by the physician by pushing the proximal end 56C from outside the body. The distal end of 56B the guide wire 56A travels through the guide wire lumen 55A, passes underneath the proximal end of the unmodified stent 60, exits the angled end of the lumen 55B, and enters the side-branch vessel 5. The main-vessel catheter 50 then is advanced distally into the main vessel until resistance is felt, caused by the stent-positioning guide wire 56A pushing up against the ostium of side-branch vessel 5. The stiffness of the stent-positioning guide wire 56A causes the main-vessel catheter 50 bearing the unmodified stent 60 to rotate so that a stent cell 62 is precisely facing the ostium of the side-branch vessel 5. The expandable member 54 is expanded by known means so that the unmodified stent 60 expands into contact with the main-vessel 6. The expandable member 54 then is deflated, and the catheter 50 is withdrawn from the patient's vascular system, leaving the guide wire 56A in the side-branch vessel 5.

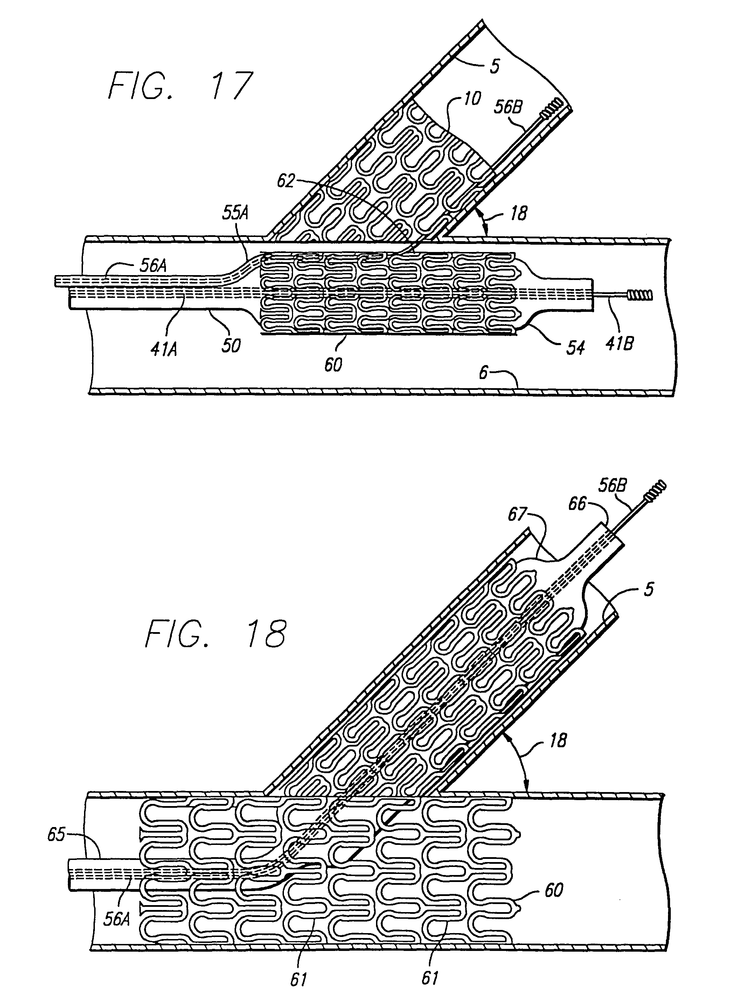

At this point, the proximal-angled stent 10 is implanted in the side-branch vessel and the unmodified main-vessel stent 60 is implanted in the main vessel and extends across the side-branch vessel 5. In order to provide an opening in the unmodified main-vessel stent 60 that aligns with the opening to the side-branch vessel, a third catheter 65, which can be a standard PTCA catheter, is back loaded onto the integrated stent-positioning guide wire 56A, already in side-branch vessel 5, and is advanced within the patient's vascular system over the guide wire. As shown in FIG. 18, the distal end 66 of the catheter 65 is advanced over the guide wire 56A until the distal end 66 of the catheter 65 begins to pass through the cell 62 of the unmodified main-vessel stent 60 and to enter the side-branch vessel 5. The catheter 65 can be of a known type used in angioplasty, as described above, having a non-distensible member or balloon 67. Once the balloon 67 is positioned through the stent cell 62 and in the opening of the side-branch vessel 5, it is expanded, thereby expanding some of the struts 61 comprising the unmodified stent 60 and forming a substantially circular opening from the main-vessel 6 through the unmodified stent 60 and into the side-branch vessel 5. In essence, the balloon 67 spreads apart some of the struts of the unmodified stent 60 to form an opening in the stent 60 that corresponds to the opening to the side-branch vessel 5, thereby providing a clear blood flow path between the main vessel and the side-branch vessel.

The unmodified main-vessel stent 60 is positioned so that it crosses the opening to the side-branch vessel 5. As is set forth above, a particularly well-suited stent for this arrangement includes a stent distributed under the tradename MultiLink® Stent, manufactured by Advanced Cardiovascular Systems, Inc., Santa Clara, California. By implanting the unmodified main-vessel stent 60 in the main-vessel 6 with an appropriate stent cell precisely aligned with the side-branch ostia, dilatation through this same cell over the guide wire 56A assures a fully expanded and non-distorted cell at the ostium of the side-vessel 5.

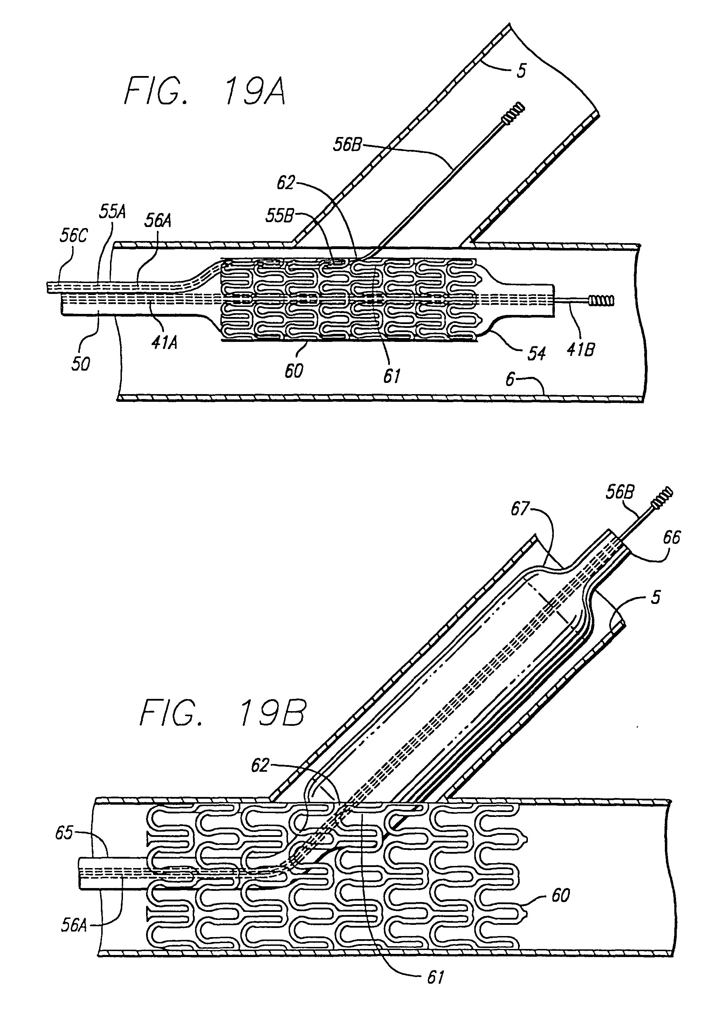

In an alternative arrangement shown in FIGS. 19A-19C, the unmodified stent 60 is implanted first, then the side-branch proximal-angled stent 10 is implanted. In one method of deploying the unmodified stent 60, the unmodified stent 60 can be mounted on the expandable member 54 of the main-vessel catheter 50. The main-vessel catheter 50 is back loaded onto the proximal end of the guide wire 41A. The main-vessel catheter 50 is advanced over the guide wire 41A and is viewed under fluoroscopy until the unmodified stent 60 is positioned in the main-vessel 6, proximally of side-branch vessel 5. The distal end of the integrated stent-positioning guide wire 56B then is advanced by the physician, who pushes the proximal end 56C from outside the patient's body. The distal end 56B of the guide wire 56A travels through the second guide wire lumen 55A, passes underneath the proximal end of the unmodified stent 60, exits the angled end of the lumen 55B and enters the side-branch vessel 5. The main-vessel catheter 50 then is advanced distally into the main vessel until resistance is felt, caused by the stent-positioning guide wire 56A pushing up against the ostium of the side-branch vessel 5. The stiffness of stent-positioning guide wire 56A causes the main-vessel catheter 50 bearing the unmodified stent 60 to rotate, so that a stent cell 62 is precisely facing the side-branch vessel 5 ostium. The expandable member 54 is expanded by known means so that the unmodified stent 60 expands into contact with the main-vessel 6. The expandable member 54 then is deflated, and the catheter 50 is withdrawn from the patient's vascular system, leaving the guide wire 56A in the side branch 5.

As shown in FIG. 19B, a third catheter 65, which can be a standard PTCA catheter, is back loaded onto the guide wire 56A, which already is in the side-branch vessel 5, and is advanced within the patient's vascular system over the guide wire. The distal end 66 of the catheter 65 is advanced over the guide wire 56A until the distal end 66 of the catheter begins to pass through the struts 61 of the stent cell 62 of the unmodified main-vessel stent 60 and then to enter the side-branch vessel 5. The catheter 65 can be of a known type used in angioplasty, as described above, having a non-distensible member or balloon 67. Once the balloon 67 is positioned through a stent cell 62 into the opening of the side-branch vessel 5, it is expanded, thereby expanding some of the struts comprising the unmodified stent 60 and forming a substantially circular opening from the main-vessel 6 through the unmodified stent 60 and into the side-branch vessel 5. In essence, the balloon 67 spreads apart the struts 61 of the unmodified stent 60 to form an opening in the unmodified stent which corresponds to the opening to the side-branch vessel 5, thereby providing a clear opening for further stenting of the side-branch vessel 5.

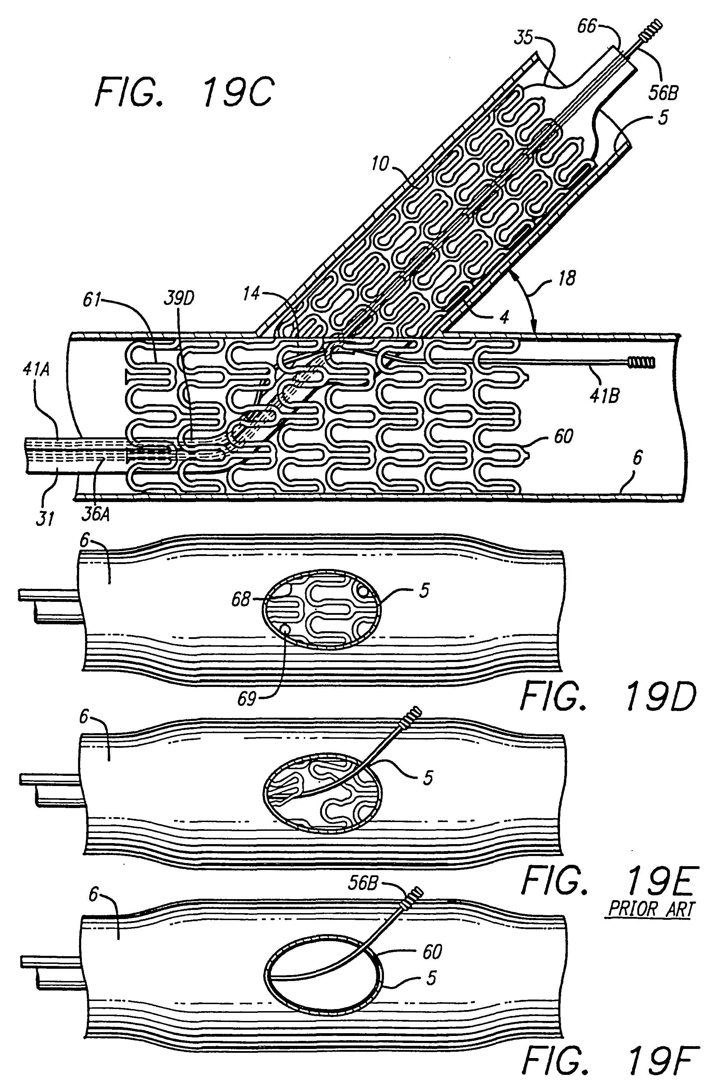

With the main vessel now stented as depicted in FIGS. 19A-19C, the side-branch vessel 5 is stented in the same manner as is described with respect to FIGS. 9-11. The only difference is that in FIG. 19, the unmodified main-vessel stent 60 already is implanted when the catheter 31 is advanced into the side-branch vessel 5. The side-branch catheter 31 is back loaded onto the tracking guide wire 36A already in the side-branch vessel 5. The side-branch catheter 31 then is advanced until the distal tip of the side-branch catheter 31 just enters the ostium of the side-branch vessel 5. The distal end 41B of the integrated stent-positioning guide wire 41A then is advanced by the physician, who pushes the proximal end 41C of the guide wire from outside the patient's body. The distal end 41B of the integrated stent-positioning guide wire 41A travels through the second guide wire lumen 39A and the angled portion 39B thereof passes close to the proximal end of the proximal-angled stent 10 and the expandable member 35, and exits the angled portion ofthe second guide wire lumen 39B. The stent-positioning guide wire 41A is advanced until the distal end 41B is distal of the side-branch vessel 5. The catheter then is advanced into the side-branch vessel until resistance is felt, caused by the stent-positioning guide wire 41 A pushing up against the ostium of the side-branch vessel. As previously described, the stent-positioning wire 41A is relatively stiff, as is the tracking guide wire 36A, so that each properly can orient the side-branch catheter 31 as it is advanced into the side-branch vessel. The angled portion 39B of the second guide wire lumen 39A is angled to assist in rotating the side-branch catheter into proper position into the side-branch vessel 5. If the stent approaches the side-branch vessel in the incorrect position, the stent-positioning wire 41A would be forced to make a very acute angle. The wire stiffness, however, prevents this from happening and causes the wire to assume the position of least stress. To relieve the buildup of stress, the guide wire 41A creates a torque on the angled portion of the second guide wire lumen 39B, causing the guide wire lumen 39A and the side-branch catheter 31 with the proximal-angled stent 10 to rotate into the correct position. Once the proximal-angled stent is positioned in the side-branch vessel 5, the expandable member 35 is expanded so that the proximal-angled stent expands into contact with the side-branch vessel 5, making sure that the proximal end 14 of the proximal-angled stent 10 covers and is aligned with the side-branch vessel 5 at the bifurcation 4. The proximal end 14 is aligned so that it coincides with the acute angle 18, thereby ensuring that all portions of the side-branch vessel 5 are covered by the proximal-angled stent, including the area where the side-branch vessel 5 meets the main-vessel 6. An unobstructed blood-flow path now exists between the expanded unmodified stent 60 and the main-vessel 6 through the opening previously formed and into the side-branch vessel 5 and through the implanted proximal-angled stent 10.

Prior art devices have generally failed that have attempted to first stent the main vessel and randomly select a stent cell to expand for alignment with the side-branch vessel. One such approach, known as the "monoclonal antibody" approach, as depicted in FIGS. 19D and 19E, shows what can happen when an inappropriate target stent cell is selected randomly and then expanded by a high pressure balloon. As shown in FIG. 19D, which is a view looking down the side-branch vessel 5, in cross-sections, at a prior art stent 68, the physician randomly selects the stent cell 69 which is a sub-optimal cell to expand with the balloon portion of a catheter. As depicted in FIG. 19E, after balloon expansion in the suboptimal cell 69, entry into the cell with a catheter may be impossible or, if accomplished, expansion of the balloon to deploy a stent in the side-branch vessel may be incomplete. The aperture created will be inadequate and major distortion in the adjacent stent struts may occur. Consequences may include subacute thrombosis or restenosis. With the arrangement shown in FIGS. 19A-19C, the target stent cell 62 is the optimal cell for expansion, and is preselected with a guide wire that is in place before stent deployment (that same wire remaining in place for subsequent access), and is oriented optimally with respect to the ostium of the side-branch vessel prior to deployment. The resulting expansion, as shown in FIG. 19F, guarantees an optimal aperture where the stent struts have been expanded, providing a blood flow path from the main vessel to the side-branch vessel.

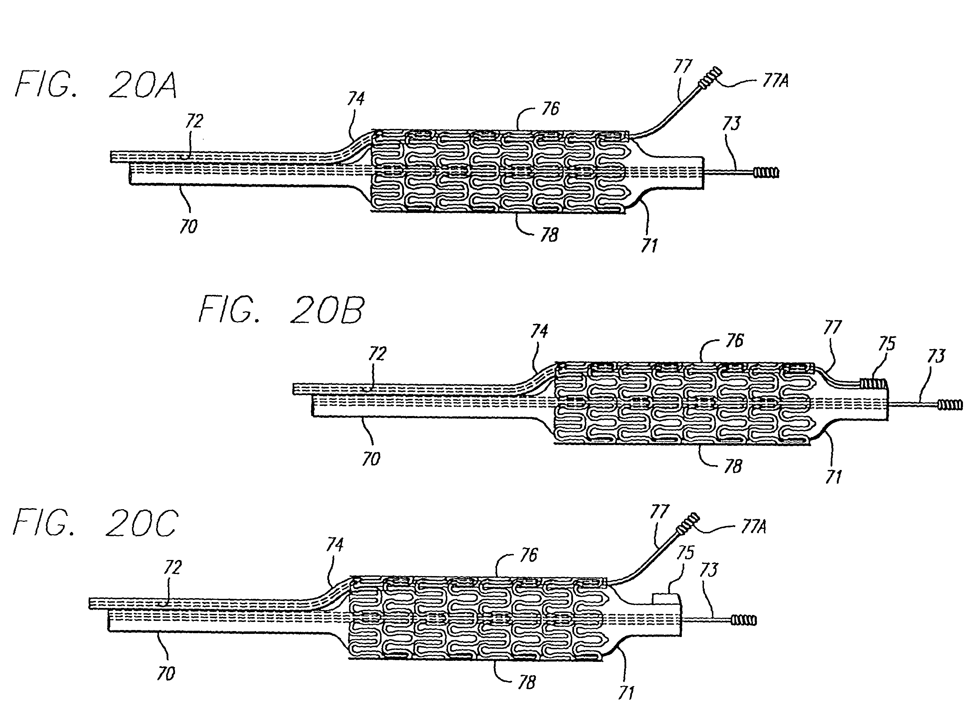

In another arrangement for stenting a bifurcation, as depicted in FIGS. 20A-20C, the main-vessel catheter 70 includes an expandable member 71 near its distal end, while the proximal end of the catheter (not shown) is similar to those previously described and either can be of the rapid-exchange or over-the-wire type. The catheter 70 includes a tracking guide wire lumen 72 for slidably receiving a tracking guide wire 73, the lumen 72 extending at least partially through the catheter in the rapid-exchange configuration, and all the way through the catheter in the over-the-wire configuration. The catheter also includes a positioning guide wire lumen 74 that is associated with the outer surface of the catheter and which is adapted to -receive positioning guide wire 77. The positioning guide wire lumen 74 extends onto and is attached to at least a portion of the expandable member 71. As shown in FIG. 20A, the positioning guide wire lumen 74 extends along the expandable member and ends just at the distal taper of the expandable member. As depicted in FIGS. 20B and 20C, the positioning guide wire lumen 74 can be formed of two sections, namely, a distal section 75 attached to the distal tip of the catheter, and a proximal section 76 extending along and attached to the expandable member and the catheter. As previously described, the two guide wires 73,77 are intended to be relatively stiff wires so that each can more easily maneuver the catheter. In these embodiments, a stent 78 is mounted on the expandable member 71 and over the positioning guide wire lumen 74. The positioning guide wire 77 is configured for slidable movement within the positioning lumen 74.

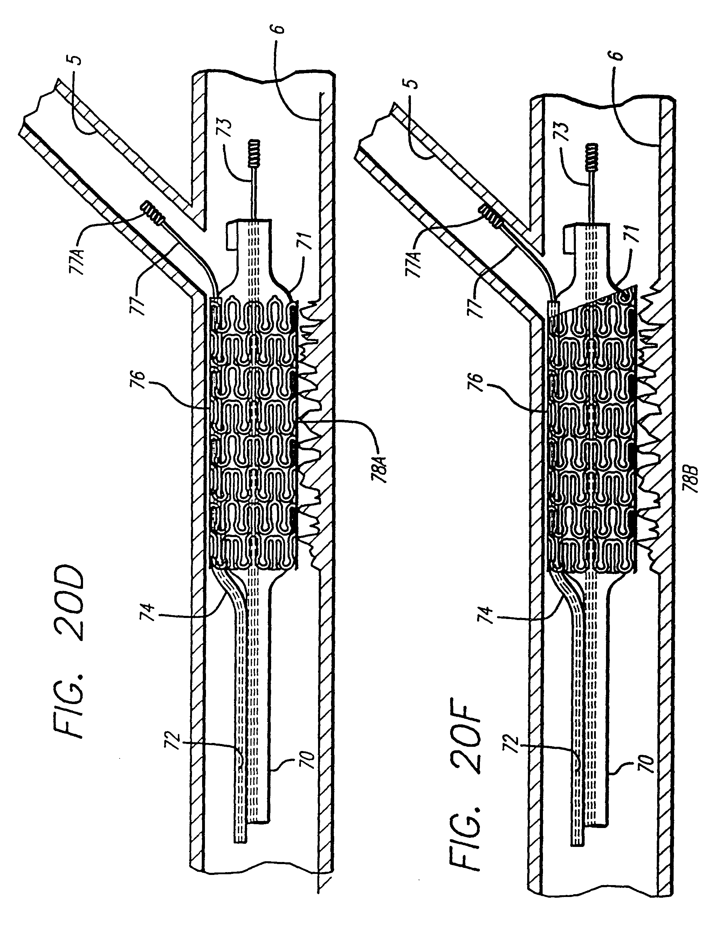

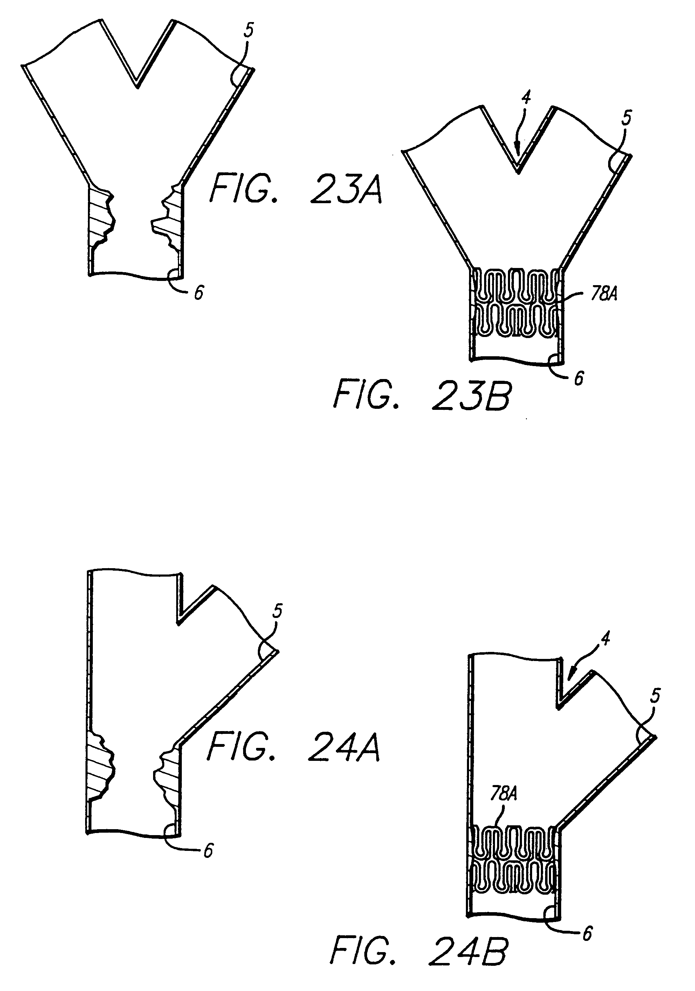

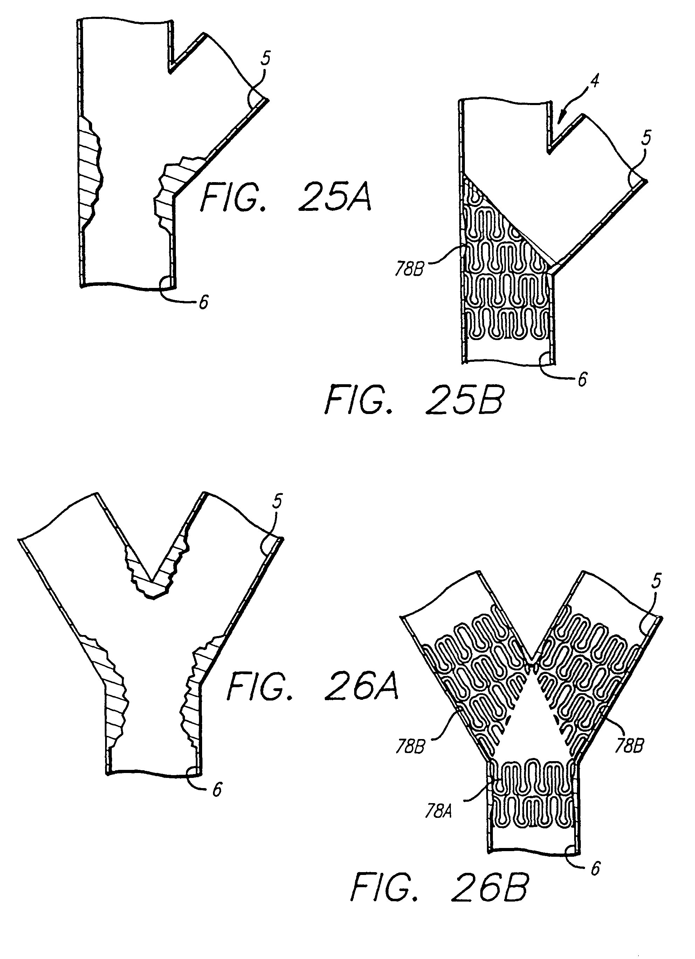

In one method of stenting a vessel just proximal to a bifurcation using the main-vessel catheter 70, the tracking guide wire 73 first is positioned within the main vessel as previously described. The catheter then is back loaded onto the guide wire by inserting the wire into the tracking guide wire lumen 72 and advancing the catheter into the patient's vascular system. At this point, the positioning guide wire 77 resides within the positioning guide wire lumen 74 and is carried into the main vessel where it will be released and advanced. Once the catheter has reached the target area, the positioning guide wire 77 is advanced distally out of the positioning guide wire lumen (for the situation illustrated in FIG. 20A) or pulled back slightly out of the distal section 75 of the positioning guide wire lumen (for the situation illustrated in FIGS. 20B and 20C). Once released by removal of the guide wire, the distal section 75 will spring out so that the positioning guide wire can seek out and be advanced into the side-branch vessel. Once the positioning guide wire is advanced in the side-branch vessel, the catheter again is advanced and the stent is implanted in the main vessel in a manner similar to that described for other arrangements. The catheter of FIGS. 20A-20C is designed to allow deployment of a stent very near but not "snowplowing" a bifurcation or side branch and is configured for treating bifurcations as depicted in FIGS. 23A-25B. A commonly encountered situation in which the catheter 70 would be used is a left anterior descending (LAD) artery that has disease right at, and proximal to, the diagonal take-off. After a careful look at multiple views, the physician should be convinced that the diagonal is spared, but the lesion is very close to and or is immediately adjacent the diagonal take-off, as shown in FIG. 20D. It is very difficult to position a standard stent in the LAD and to be certain that the lesion is fully covered and the diagonal is not snowplowed or jailed. The catheter 70, having one wire in the LAD (main vessel) and the other in the diagonal (side-branch vessel), would allow precise definition of the bifurcation and would avoid these problems. A square stent 78A, which has both ends transverse to the stent axis, could be deployed just proximal to the carina, in which case the stent distal end may need to be flared a bit, or more likely, relaxed back to where the positioning guide wire 77 is resting against the proximal aspect of the ostium, visually defining the ostium in relationship to the stent and allowing precise deployment.

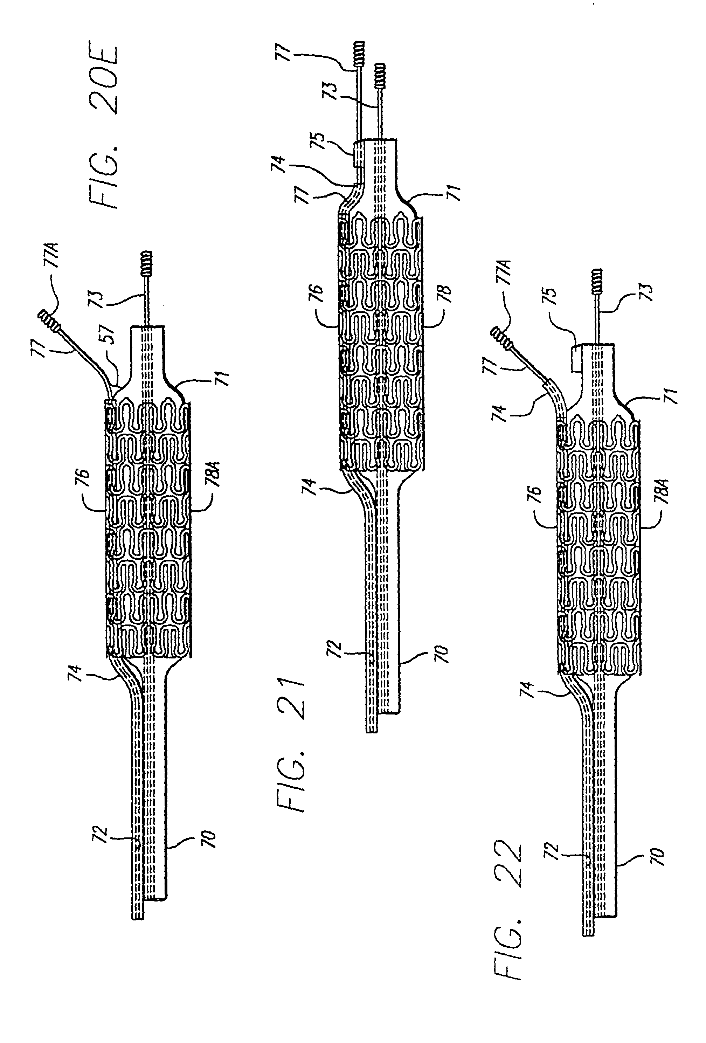

Several alternative arrangements of the main-vessel catheter 70 shown in FIG. 20A, are depicted in FIGS. 20E, 21 and 22. The catheter device shown in FIG. 20E is similar to that shown in FIG. 20A, with the exception that the ramp 57 is employed just distally of the distal end of the guide wire lumen 74 so that as the guide wire 77 exits the lumen, it will move outwardly along the ramp 57 so that it more easily advances into the side-branch vessel. Likewise, as shown in FIGS. 21 and 22, which are similar to the catheter described and depicted in FIGS. 20B and 20C, it is intended that the guide wire 77 move outwardly so that it can more easily be advanced into the side-branch vessel 5. In that regard, the distal end of the guide wire lumen 74 is biased outwardly as shown in FIG. 22, so that as the guide wire 77 is pulled back from the lumen 75, the distal end of guide wire lumen 74 will spring outwardly thereby assisting the guide wire 77 in moving radially outwardly to be positioned in the side-branch vessel.

In order to implant a square main-vessel stent 78A in a main vessel, where the disease is at or just proximal to the side-branch vessel, the catheter 70 as depicted in FIGS. 21 and 22 is well suited. For example, the catheter 70 is advanced over the guide wire 77 until the catheter is positioned just proximal of the side-branch vessel. The tracking guide wire 73, which up to this point has been contained within the main-vessel catheter 70, is advanced into the main vessel so that it is distal of the side-branch vessel. The guide wire 77 then is withdrawn proximally so that its distal end 77A is withdrawn from the distal section 75 of the positioning guide wire lumen 74, whereupon the guide wire 77 and the distal end of the guide wire lumen 74 spring outwardly, thereby assisting the positioning of the guide wire 77 into the side-branch vessel. The guide wire 77 then is advanced into the side-branch vessel and the main-vessel catheter 70 is advanced so that the guide wire 77 rests on the proximal ostium of the side-branch vessel, wherein the square stent 78A then can be expanded to cover the diseased portion, but not to span or cover (i.e., "jail") the opening to the side-branch vessel.

If the diseased portion of a main vessel is directly adjacent the opening to the side-branch vessel, as depicted in FIG. 20F then the catheter system as depicted in FIG. 20A can be incorporated but to implant a distal-angled stent 78B rather than square stent 78A. As shown in FIG. 20F, the stent 78B has an angle at its distal end which coincides with the opening to the side-branch vessel so that the diseased portion of the main vessel is covered by the distal end of the stent, with the angle of the stent angled proximally so that the side-branch vessel is not covered or jailed. Various alternatives of the square stent 78A and the distal-angled stent 78B can be used for treating various conditions as depicted in FIGS. 23A through 26B.

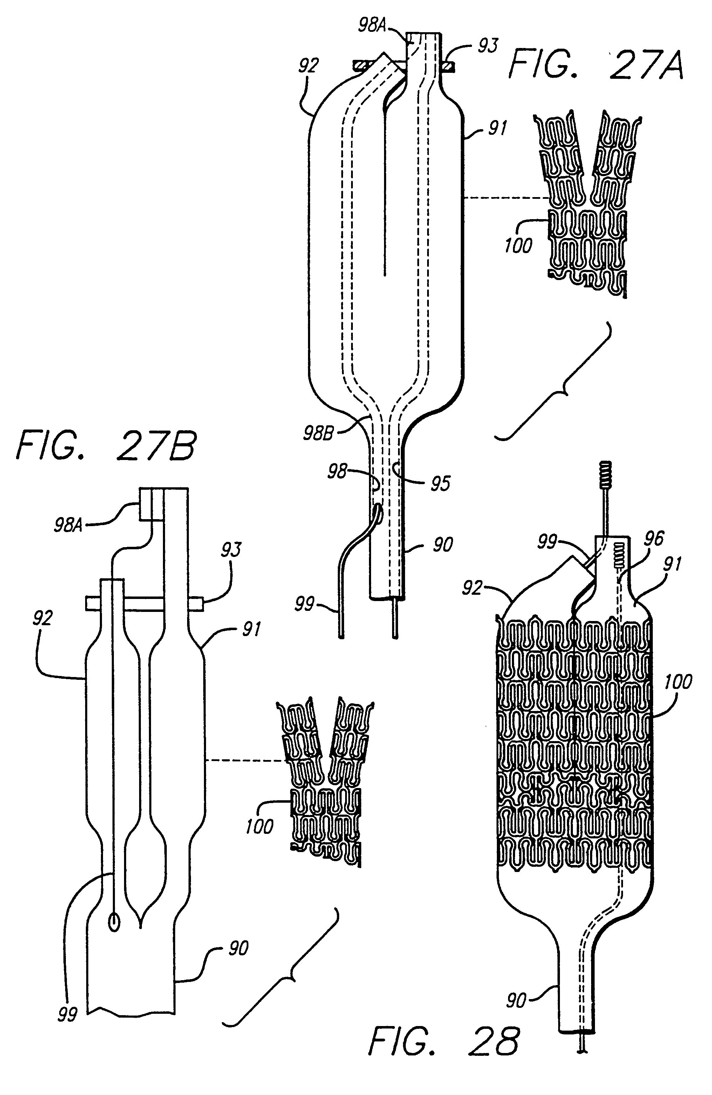

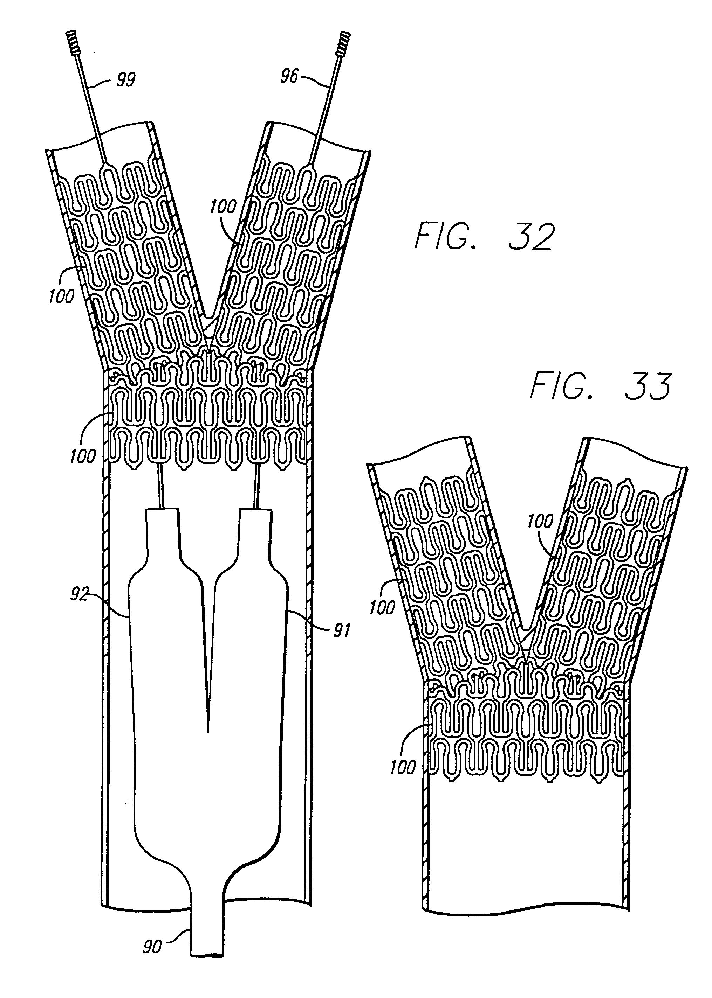

In another arrangement depicted in FIGS. 27-33, a dual balloon Y-shaped catheter assembly is provided to stent a bifurcation. In this arrangement, a Y-shaped stent is implanted to cover the bifurcation. The catheter 90 includes first and second expandable members 91,92 that are configured to reside side by side (Y-shaped) for low profile delivery and to spring apart for implanting the stents. A locking ring 93 may be used to assist in holding the expandable members together until just prior to deployment, at which time the locking ring can be removed. A guide wire lumen 95 extends at least through a portion of the catheter and slidably receives a guide wire 96. The second guide wire lumen 98 extends at least through a portion ofthe catheter and slidably receives the second guide wire 99. The second guide wire lumen 98 includes distal section 98A and 98B. A Y-shaped stent 100 is mounted on the first and second expandable members 91, 92.

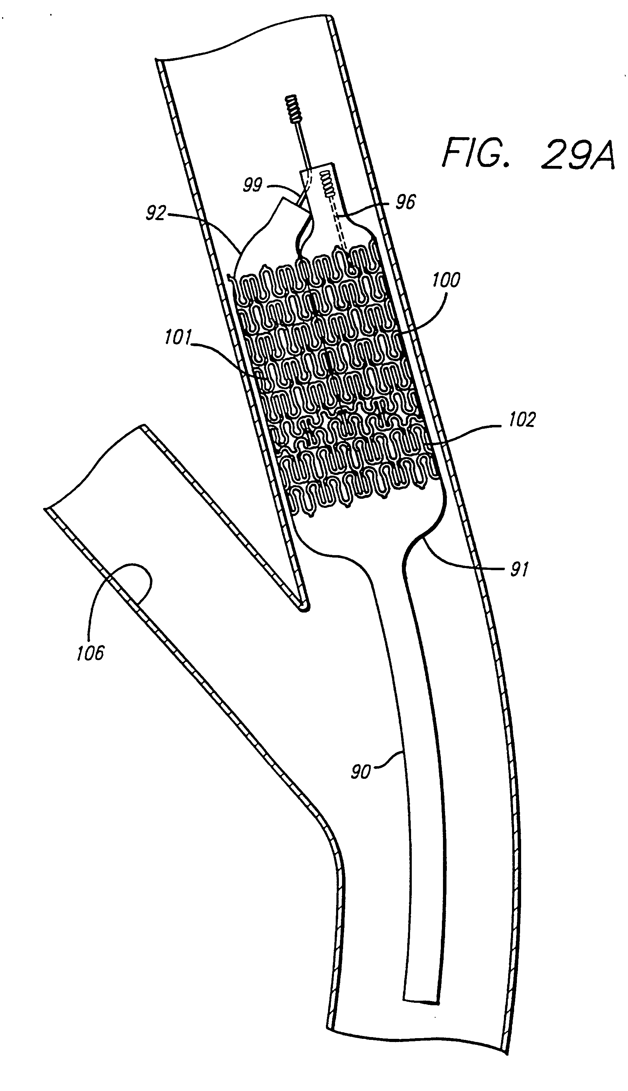

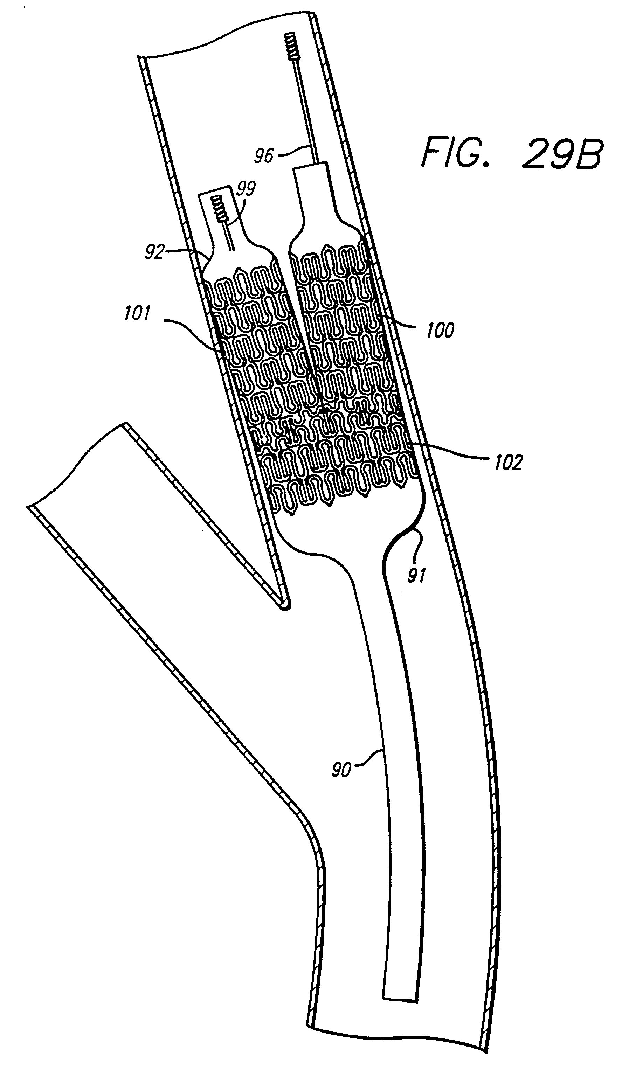

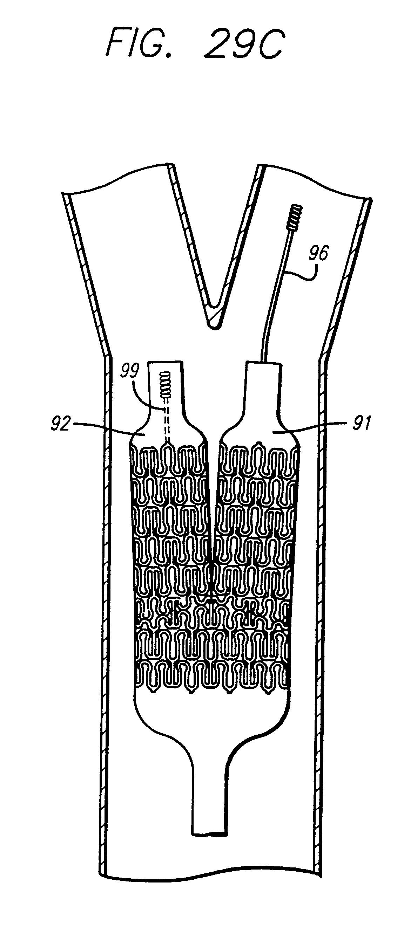

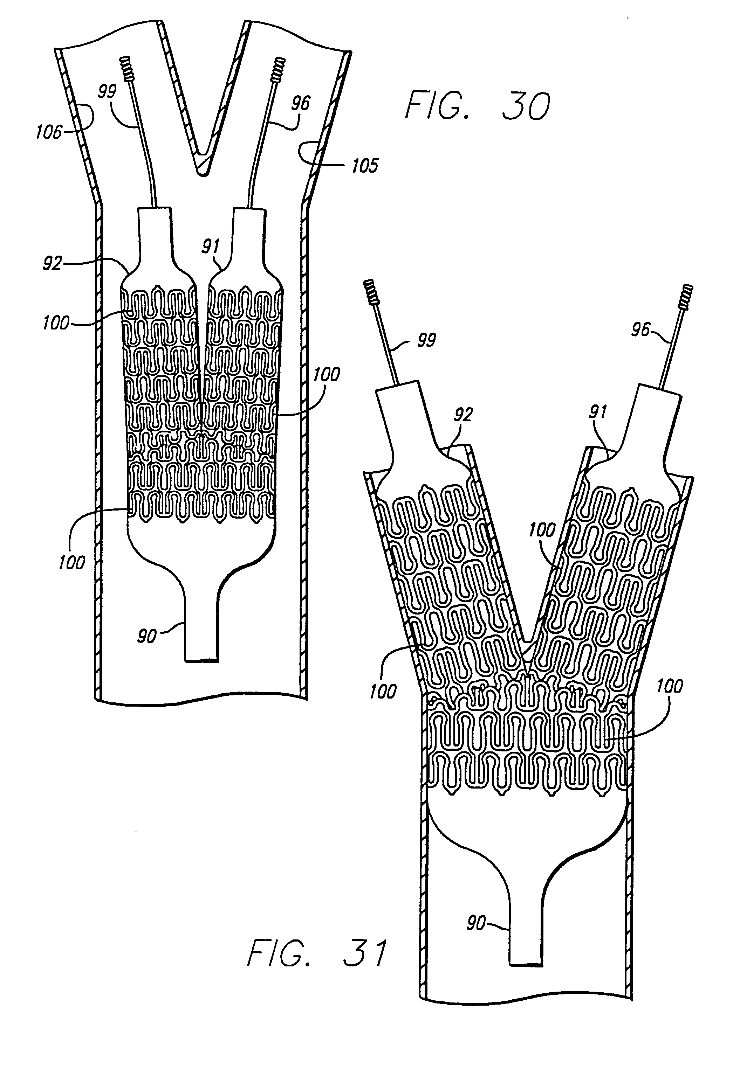

In the method of stenting the bifurcated vessels shown in FIGS. 29 to 33, the second guide wire 99, previously positioned distal to the bifurcation in one limb (perhaps the limb most vulnerable to problems for wire re-crossing), is back loaded into the lumens 98A and 98B and the catheter 90 is advanced over the second guide wire 99 so that the catheter is advanced distally beyond the bifurcation. The guide wire 96, which has been contained in the lumen 95 to this point, is advanced along the side of the guide wire 99. The guide wire 99 then is withdrawn until its distal end pulls out of the distal section 98A of the second guide wire lumen 98. As the guide wire 99 is pulled back (proximally), the first and second expandable members 91,92, which normally are biased together, are released and now spring apart. The guide wire the guide wire lumen for which is most distant (lateral) to the bifurcation (in this case the guide wire 96) then is advanced into the distal vessel and the other guide wire (in this case the second guide wire 99) is withdrawn as illustrated in FIG. 29B. The catheter then is withdrawn proximally so that the expandable members 91, 92 are now proximal to the bifurcation as depicted in FIG. 29C and the other guide wire (in this case, the second guide wire 99) is advanced into the other limb of the bifurcation as shown in FIG. 30. The catheter 90 then is advanced distally over both guide wires 96 and 99, as shown in FIG. 31, until the stent 100 is positioned in the bifurcation of the intersection of vessels 105,106. Due to the appropriate wire selection, rotation of no more than ninety degrees (90°) will be required. A stent 100 is implanted by inflating the expandable members 91,92 in a known manner. The expandable members then are deflated, and the catheter is withdrawn from the patient. The arrangement of the guide wires 96 and 99 and the respective lumens of each permit single unit transport of a Y-stent to the distal target site without wire wrapping problems and further allows for minimal requirements of rotation of the device (less than ninety degrees (90°)) for optimal deployment (allowing minimal twist deformity). The guide wires may be left in place for further intervention such as for finishing the stents with simultaneous high pressure balloon inflation.

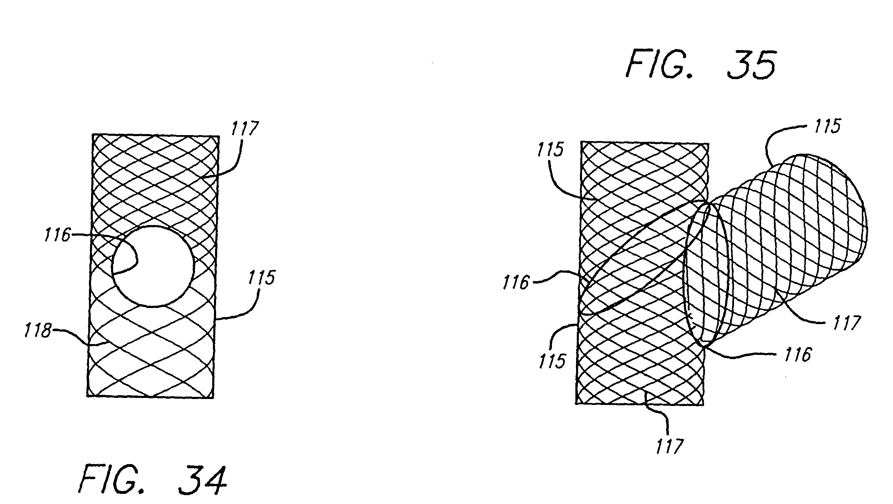

In another arrangement, a pair of stents as depicted in FIGS. 34-36 having varying stent cell density are implanted in a bifurcated vessel, as depicted in FIGS. 34-36C.

As shown in FIG. 34, an apertured stent 115 is provided in which an aperture 116 is positioned on its outer surface. The stent 115 includes areas of heavy stent cell density 117 and areas of light stent cell density 118 along its outer surface. As can be seen in FIG. 35, two stents 115 have been combined so that the areas of light stent cell density 118 of one overlaps the areas of light cell stent density 118 of the other causing the combined stents to create relatively uniform heavy cell density 117 and thus providing relatively uniform heavy cell density over the entire bifurcated vessel wall.

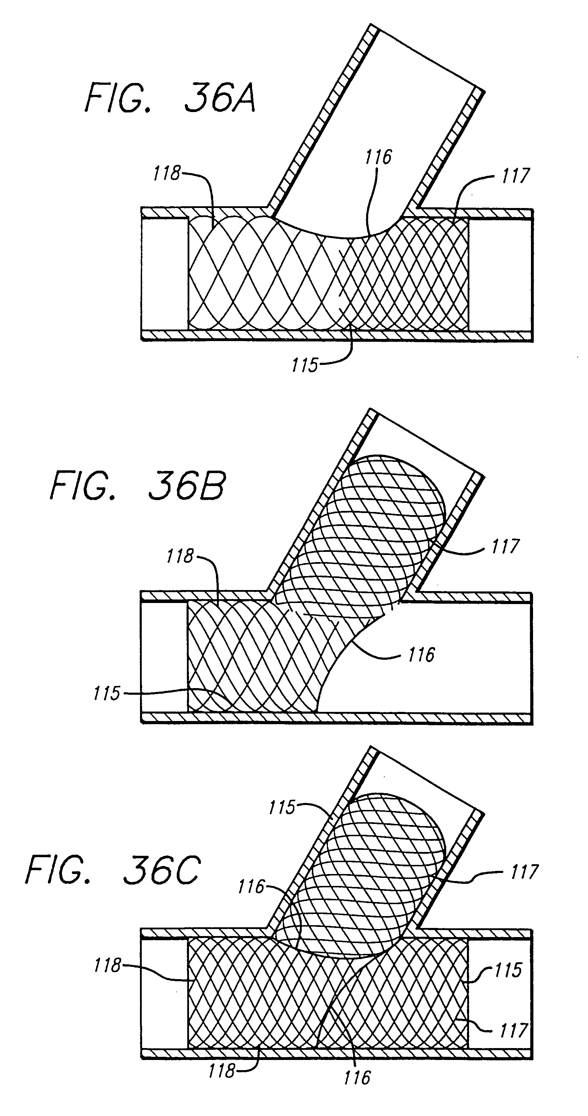

As shown in FIGS. 36A to 36C, two stents 115 are implanted to stent the bifurcation. For the sake of clarity, as shown in FIG. 36A, the apertured stent 115 is shown implanted in the main vessel such that the aperture 116 spans and provides an opening to the side-branch vessel, while the areas of heavy stent cell density 117 provide full coverage of the distal main vessel by the stent 115. As depicted in FIG. 36B, the apertured stent 115 is partially implanted in the side-branch vessel and partially implanted in the main vessel, in this case with the aperture 116 facing the continuing lumen of the main vessel. More specifically, the areas of heavy stent cell density 117 are implanted in the side-branch vessel, while the areas of light stent cell density 118 are implanted in the main vessel, with the aperture 116 providing an opening for blood flow through the main vessel. It is intended that the apertured stent 115 be implanted first as seen in FIG. 36A and that a second apertured stent 115 subsequently be implanted as shown in FIG. 36B or, according to physician preference, this sequence may be reversed. Thus, in FIG. 36C, both apertured stents 115 have been implanted, and both apertures 116 provide openings so that blood flow is unimpaired through both the main vessel and the side-branch vessel, and no stent struts are left unapposed. The areas of light stent cell density 118 of both the apertured stents 115 overlap proximal to the bifurcation, thereby insuring that there is full coverage of the bifurcated area by heavy stent cell density. Both apertured stents 115 are implanted with the catheter delivery system described herein, which includes a positioning wire to accurately position and implant the stents in the bifurcated vessels.

While the invention herein has been illustrated and described in terms of an assembly for stenting bifurcated vessels, it will be apparent to those skilled in the art that the stents and delivery systems herein can be used in the coronary arteries, veins and other arteries throughout the patient's vascular system. Certain dimensions and materials of manufacture have been described herein, and can be modified without departing from the scope of the invention.

## Claims

1. A stent delivery assembly for treating bifurcated vessels having a side-branch vessel (5) and a main vessel (6), comprising:
a side-branch catheter (31) having a proximal end (33) and a distal end (32);
an expandable member (35) proximate the distal end (32) of the catheter (31);
a tracking guide wire lumen (34A) extending within at least a portion of the catheter (31);
a tracking guide wire (36A) having a distal end (36B) and a proximal end (36C) and sized for slidable movement within the tracking guide wire lumen (34A),
**characterized by** a positioning guide wire lumen (39A) having an angulated section (39B) and being firmly attached to an outer surface of the catheter (31) and associated with the expandable member (35) in both its unexpanded and fully expanded condition and adapted to receive for slidable engagement a positioning guide wire (41A), the positioning guide wire having a distal end (41B) and a proximal end (41C), the proximal ends (36C,41C) of the tracking and positioning guide wires (36A,41A) extending out of the patient and manipulatable so that the distal end (41B) of the positioning guide wire (41A) is advanced in the main vessel (6) distal to the side-branch vessel (5), and the distal end (36B) of the tracking guide wire (36A) is advanced into the side-branch vessel (5).

2. The stent delivery assembly of claim 1, wherein the positioning guide wire lumen (39A) is attached to an outer surface (40) of the catheter (31) and extends along to just proximal of the expandable member (35).

3. The stent delivery assembly of claim 1 or claim 2, wherein the positioning guide wire lumen (39A) includes a straight portion (39C).

4. The stent delivery assembly of claim 3, wherein the angulated portion (39B) is at an angle relative to the straight portion (39C), and the angle is within the range of angles from five degrees (5°) to ninety degrees (90°).

5. The stent delivery assembly of any preceding claim, wherein a stent (10) is mounted on the expandable member (35), the stent (10) including an angled proximal end (14) for mounting on the expandable member (35) and for deployment in the side-branch vessel (5).

6. The stent delivery assembly of any preceding claim, wherein a side-branch vessel stent (10) is removably mounted on the expandable member (35) and configured for implanting in the side-branch vessel (5).

7. The stent delivery assembly of claim 6, further comprising a main-vessel catheter (50) having a distal end (51) and a proximal end (52) and having a tracking guide wire lumen (53A) extending through at least a portion thereof, the tracking guide wire lumen (53A) of the main-vessel catheter (50) being sized for receiving the tracking guide wire (36A) for slidable movement therein; an expandable member (54) positioned near the main-vessel catheter (50) distal end (51) for delivering and implanting a main-vessel stent (20) adjacent to the side-branch vessel stent (10); and a positioning guide wire lumen (55A) attached to the outer surface of the main-vessel catheter (50) and extending over at least a portion of the surface of the expandable member (54) and sized for slidably receiving the positioning guide wire (56A), the positioning guide wire lumen (55A) advancing over the positioning guide wire (56A) to orient the expandable member (54) adjacent to, but not in, the side-branch vessel (5).

8. The stent delivery assembly of any preceding claim, wherein the positioning guide wire (56A) comprises an integrated stent-positioning guide wire (41A or 56A) for accurately positioning a stent (10 or 20), the side-branch catheter (31) being configured for rapid exchange so that the catheter (31) can be unzipped from the integrated stent-positioning guide wire (41A) leaving the guide wire (41A) in place for additional interventions.

9. The stent delivery assembly of any preceding claim, wherein the expandable member (35 or 54) includes an angled proximal taper balloon for deploying a proximal-angled stent (10) at the bifurcation site.

10. The stent delivery assembly of any preceding claim, wherein the side-branch catheter (31) comprises a rapid exchange catheter and includes a distal end opening (34B) in the tracking guide wire lumen (34A) and a side port opening (34C) on an outer surface of the side-branch catheter (31) so that the tracking guide wire (36A) extends through the side port opening (34C), through the tracking guide wire lumen (34A), and out the distal end opening (34B), the catheter (31) further including a slit extending from the side port opening (34C) to just proximal of the expandable member (35 or 54) so that the tracking guide wire (36A) can be unzipped through the slit during catheter exchanges.

11. The stent delivery assembly of any preceding claim, wherein the side-branch catheter (31) comprises an over-the-wire catheter and includes a distal end opening in the tracking guide wire lumen (34A) and a proximal opening in the tracking guide wire (34A) lumen so that the tracking guide wire (36A) extends from outside the proximal end opening, through the tracking guide wire lumen (34A), and out the distal end opening.

12. The stent delivery assembly of claim 7, wherein the main-vessel catheter (50) is a rapid exchange catheter and includes a distal end opening in the tracking guide wire lumen (53A) and a side port opening (53C) on an outer surface of the main-vessel catheter (50) so that the tracking guide wire (36A) extends through the side port opening (53C) on the outer surface of the main-vessel catheter (50), through the tracking guide wire lumen (53A), and out the distal end opening of the main-vessel catheter (50), the main-vessel catheter (50) further including a slit extending from the side port opening (53C) so that the tracking guide wire (36A) can be pulled through the slit during a catheter exchange.

13. The stent delivery assembly of claim 7, wherein the main-vessel catheter (50) is an over-the-wire catheter and includes a distal end opening in the tracking guide wire lumen (53A) and a proximal opening in the tracking guide wire lumen (53A) so that the tracking guide wire (34A) extends from outside the proximal end opening, through the tracking guide wire lumen (53A) and out the distal end opening.

## Patentansprüche

1. Stenteinführanordnung zum Behandeln verzweigter Gefäße, die ein Seitenastgefäß (5) und ein Hauptgefäß (6) aufweisen, umfassend:
einen Seitenastkatheter (31), der ein proximales Ende (33) und ein distales Ende (32) aufweist;
ein aufweitbares Element (35) proximal des distalen Endes (32) des Katheters (31);
ein Spurführungsdraht-Lumen (34A), das sich in zumindest einem Abschnitt des Katheters (31) erstreckt;
einen Spurführungsdraht (36A), der ein distales Ende (36B) und ein proximales Ende (36C) aufweist und zur Gleitbewegung in dem Spurführungsdrahtlumen (34A) bemessen ist,
**gekennzeichnet durch** ein Positionierungsführungsdrahtlumen (39A), das einen gewinkelten Abschnitt (39B) aufweist und fest an einer Außenfläche des Katheters (31) angebracht ist und dem aufweitbaren Element (35) sowohl in dessen nicht aufgeweiteten als auch dessen vollständig aufgeweiteten Zustand zugeordnet und ausgelegt ist, zum verschiebbaren Eingriff einen Positionierungsführungsdraht (41A) aufzunehmen, wobei der Positionierungsführungsdraht ein distales Ende (41B) und ein proximales Ende (41C) aufweist, wobei sich die proximalen Enden (36C, 41C) der Spurführungs- und
Positionierungsführungsdrähte (36A, 41A) aus dem Patienten erstrecken und derart manipulierbar sind, dass das distale Ende (41B) des Positionierungsführungsdrahts (41A) in dem Hauptgefäß (6) distal zu dem Seitenastgefäß (5) vorgeschoben wird und das distale Ende (36B) des Spurführungsdrahts (36A) in das Seitenastgefäß (5) vorgeschoben wird.

2. Stenteinführanordnung nach Anspruch 1, worin das Positionierungsführungsdrahtlumen (39A) an einer Außenfläche (40) des Katheters (31) angebracht ist und sich entlang bis direkt proximal des aufweitbaren Elements (35) erstreckt.

3. Stenteinführanordnung nach Anspruch 1 oder Anspruch 2, worin das Positionierungsführungsdrahtlumen (39A) einen geraden Abschnitt (39C) enthält.

4. Stenteinführanordnung nach Anspruch 3, worin der gewinkelte Abschnitt (39B) einen Winkel relativ zu dem geraden Abschnitt (39C) hat, und der Winkel innerhalb des Winkelbereichs von fünf Grad (5°) bis neunzig Grad (90°) liegt

5. Stenteinführanordnung nach einem der vorhergehenden Ansprüche, worin an dem aufweitbaren Element (35) ein Stent (10) angebracht ist, wobei der Stent (10) ein gewinkeltes proximales Ende (14) zum Anbringen an dem aufweitbaren Element (35) und zum Entfalten in dem Seitenastgefäß (5) aufweist.

6. Stenteinführanordnung nach einem der vorhergehenden Ansprüche, worin ein Seitenastgefäßstent (10) an dem aufweitbaren Element (35) entfernbar angebracht ist und zum Implantieren in das Seitenastgefäß (5) konfiguriert ist.

7. Stenteinführanordnung nach Anspruch 6, ferner umfassend: einen Hauptgefäßkatheter (50) mit einem distalen Ende (51) und einem proximalen Ende (52) und mit einem Spurführungsdrahtlumen (53A), das sich durch zumindest einen Abschnitt davon erstreckt, wobei das Spurführungsdrahtlumen (53A) des Hauptgefäßkatheters (50) bemessen ist, um den Spurführungsdraht (36A) darin gleitbeweglich aufzunehmen; ein aufweitbares Element (54), das nahe dem distalen Ende (51) des Hauptgefäßkatheters (50) positioniert ist, um einen Hauptgefäßstent (20) benachbart dem Seitenastgefäßstent (10) einzuführen und zu implantieren; und ein Positionierungsführungsdrahtlumen (55A), das an der Außenfläche des Hauptgefäßkatheters (50) angebracht ist und sich über zumindest einen Abschnitt der Oberfläche des aufweitbaren Elements (54) erstreckt und bemessen ist, um den Positionierungsführungsdraht (56A) gleitbeweglich aufzunehmen, wobei sich das Positionierungsführungsdrahtlumen (55A) über den Positionierungsführungsdraht (56A) vorschiebt, um das aufweitbare Element (54) benachbart, jedoch nicht in, dem Seitenastgefäß (5) zu orientieren.

8. Stenteinführanordnung nach einem der vorhergehenden Ansprüche, worin der Positionierungsführungsdraht (56A) einen integrierten Stentpositionierungsführungsdraht (41A oder 56A) zum akkuraten Positionieren eines Stents (10 oder 20) aufweist, wobei der Seitenastkatheter (31) für eine Schnellwechsel konfiguriert ist, sodass der Katheter (31) aus dem integrierten Stentpositionierungsführungsdraht (41A) gezogen werden kann, wobei der Führungsdraht (41A) für zusätzliche Interventionen am Ort verbleibt.

9. Stenteinführanordnung nach einem der vorhergehenden Ansprüche, worin das aufweitbare Element (35 oder 54) einen gewinkelten proximal verjüngten Ballon enthält, um einen proximal gewinkelten Stent (10) an der Verzweigungsstelle zu entfalten.

10. Stenteinführanordnung nach einem der vorhergehenden Ansprüche, worin der Seitenastkatheter (31) einen Schnellwechselkatheter aufweist und eine distale Endöffnung (348) in dem Spurführungsdrahtlumen (34A) und eine Seitenlochöffnung (34C) an einer Außenfläche des Seitenastkatheters (31) enthält, sodass sich der Spurführungsdraht (36A) durch die Seitenlochöffnung (34C), durch das Spurführungsdrahtlumen (34A) und aus der distalen Endöffnung (34B) hinaus erstreckt, wobei der Katheter (31) ferner einen Schlitz enthält, der sich von der Seitenlochöffnung (34C) bis direkt proximal des aufweitbaren Elements (35 oder 54) erstreckt, sodass während Katheterwechseln der Spurführungsdraht (36A) durch den Schlitz abgezogen werden kann.

11. Stenteinführanordnung nach einem der vorhergehenden Ansprüche, worin der Seitenastkatheter (31) einen Über-dem-Draht-Katheter aufweist und eine distale Endöffnung in dem Spurführungsdrahtlumen (34A) und eine proximale Öffnung in dem Spurführungsdrahtlumen (34A) enthält, sodass sich der Spurführungsdraht (36A) von der Außenseite der proximalen Endöffnung durch das Spurführungsdrahtlumen (34A) und aus der distalen Endöffnung hinaus erstreckt.

12. Stenteinführanordnung nach Anspruch 7, worin der Hauptgefäßkatheter (50) ein Schnellwechselkatheter ist und eine distale Endöffnung in dem Spurführungsdrahtlumen (53A) und eine Seitenlochöffnung (53C) an einer Außenfläche des Hauptgefäßkatheters (50) enthält, sodass sich der Spurführungsdraht (36A) durch die Seitenlochöffnung (53C) an der Außenfläche des Hauptgefäßkatheters (50), durch das Spurführungsdraht-Lumen (53A) und aus der distalen Endöffnung des Hauptgefäßkatheters (50) erstreckt, wobei der Hauptgefäßkatheter (50) ferner einen Schlitz enthält, der sich von der Seitenlochöffnung (53C) erstreckt, sodass während eines Katheterwechsels der Spurführungsdraht (36A) durch den Schlitz gezogen werden kann.

13. Stenteinführanordnung nach Anspruch 7, worin der Hauptgefäßkatheter (50) ein Über-dem-Draht-Katheter ist und eine distale Endöffnung in dem Spurführungsdrahtlumen (53A) und eine proximale Öffnung in dem Spurführungsdrahtlumen (53A) enthält, sodass sich der Spurführungsdraht (34A) von der Außenseite der proximalen Endöffnung durch das Spurführungsdrahtlumen (53A) und aus der distalen Endöffnung hinaus erstreckt.

## Revendications

1. Ensemble d'introduction d'extenseur destiné à traiter les vaisseaux bitronculaires comportant un vaisseau de ramification latérale (5) et un vaisseau principal (6), comprenant :
un cathéter de ramification latérale (31) ayant une extrémité proximale (33) et une extrémité distale (32) ;
un élément dilatable (35) à proximité de l'extrémité distale (32) du cathéter (31);
une lumière de fil de guidage suiveur (34A) s'étendant à l'intérieur d'au moins une partie du cathéter (31);
un fil de guidage suiveur (36A) ayant une extrémité distale (36B) et une extrémité proximale (36C) et dimensionné pour un mouvement, avec faculté de glissement, à l'intérieur de la lumière du fil de guidage suiveur (34A), **caractérisé par**
une lumière de fil de guidage de positionnement (39A) comportant une section angulaire (39B) et étant fermement fixée à une surface extérieure du cathéter (31) et associée à l'élément dilatable (35) dans à la fois ses conditions non dilatée et pleinement dilatée et adaptée pour recevoir, pour mise en prise avec faculté de glissement, un fil de guidage de positionnement (41A), le fil de guidage de positionnement ayant une extrémité distale (41B) et une extrémité proximale (41C), les extrémités proximales (36C, 41C) des fils de guidage suiveur et de positionnement (36A, 41A) s'étendant hors du patient et pouvant être manipulées de sorte que l'extrémité distale (41B) du fil de guidage de positionnement (41A) est avancée dans le vaisseau principal (6) distal au vaisseau de ramification latérale (5), et l'extrémité distale (36B) du fil de guidage suiveur (36A) est avancée dans le vaisseau de ramification latérale (5).

2. Ensemble d'introduction d'extenseur selon la revendication 1, dans lequel la lumière de fil de guidage de positionnement (39A) est attachée à une surface extérieure (40) du cathéter (31) et s'étend suivant, juste proximale à, l'élément dilatable (35).

3. Ensemble d'introduction d'extenseur selon la revendication 1 ou 2, dans lequel la lumière du fil de guidage de positionnement (39A) comprend une partie droite (39C).

4. Ensemble d'introduction d'extenseur selon la revendication 3, dans lequel la partie angulaire (39B) se trouve à un angle par rapport à la partie droite (39C) et l'angle se situe dans la gamme des angles de cinq degrés (5°) à quatre-vingt-dix degrés (90°).

5. Ensemble d'introduction d'extenseur selon l'une quelconque des revendications précédentes, dans lequel un extenseur (10) est monté sur l'élément dilatable (35), l'extenseur (10) comprenant une extrémité proximale angulaire (14) pour montage sur l'élément dilatable (35) et pour déploiement dans le vaisseau de ramification latérale (5).

6. Ensemble d'introduction d'extenseur selon l'une quelconque des revendications précédentes, dans lequel un extenseur de vaisseau de ramification latérale (10) est monté, de manière amovible, sur l'élément dilatable (35) et configuré pour implantation dans le vaisseau de ramification latérale (5).

7. Ensemble d'introduction d'extenseur selon la revendication 6, comprenant, en outre, un cathéter de vaisseau principal (50) comportant une extrémité distale (51) et une extrémité proximale (52) et comportant une lumière du fil de guidage suiveur (53A) s'étendant à travers au moins l'une de ses parties, la lumière du fil de guidage suiveur (53A) du cathéter de vaisseau principal (50) étant dimensionnée pour recevoir le fil de guidage suiveur (36A) pour un déplacement, avec faculté de glissement, dans celui-ci ; un élément dilatable (54) positionné proche de l'extrémité distale (51) du cathéter de vaisseau principal (50) afin d'introduire et d'implanter un extenseur de vaisseau principal (20) adjacent à l'extenseur de vaisseau de ramification latérale (10) ; et une lumière du fil de guidage de positionnement (55A) attachée à la surface extérieure du cathéter de vaisseau principal (50) et s'étendant sur au moins une partie de la surface de l'élément dilatable (54) et dimensionnée pour recevoir, avec faculté de glissement, le fil de guidage de positionnement (56A), la lumière du fil de guidage de positionnement (55A) avançant sur le fil de guidage de positionnement (56A) pour orienter l'élément dilatable (54) adjacent à, mais non dans, le vaisseau de ramification latérale (5).

8. Ensemble d'introduction d'extenseur selon l'une quelconque des revendications précédentes, dans lequel le fil de guidage de positionnement (56A) comprend un fil de guidage de positionnement d'extenseur intégré (41A ou 56A) destiné au positionnement précis d'un extenseur (10 ou 20), le cathéter de ramification latérale (31) étant configuré pour un échange rapide de sorte que le cathéter (31) puisse être non remonté à partir du fil de guidage de positionnement d'extenseur intégré (41A), laissant le fil de guidage (41A) en place pour des interventions supplémentaires.

9. Ensemble d'introduction d'extenseur selon l'une quelconque des revendications précédentes, dans lequel l'élément dilatable (35 ou 54) comprend un ballon conique proximal angulaire pour déploiement d'un extenseur à angle proximal (10) au niveau du site de bifurcation.

10. Ensemble d'introduction d'extenseur selon l'une quelconque des revendications précédentes, dans lequel le cathéter de ramification latérale (31) comprend un cathéter d'échange rapide et comprend une ouverture d'extrémité distale (34B) dans la lumière du fil de guidage suiveur (34A) et une ouverture d'orifice latérale (34C) sur une surface extérieure du cathéter de ramification latérale (31) de sorte que le fil de guidage suiveur (36A) s'étende à travers l'ouverture d'orifice latérale (34C), à travers la lumière du fil de guidage suiveur (34A), et hors de l'ouverture de l'extrémité distale (34B), le cathéter (31) comprenant, en outre, une fente s'étendant de l'ouverture de l'orifice latérale (34C) vers la proximité de l'élément dilatable (35 ou 54) de sorte que le fil de guidage suiveur (36A) puisse être non remonté à travers la fente pendant les échanges de cathéter.

11. Ensemble d'introduction d'extenseur selon l'une quelconque des revendications précédentes, dans lequel le cathéter de ramification latérale (31) comprend un cathéter sur le fil et comporte une ouverture d'extrémité distale dans la lumière du fil de guidage suiveur (34A) et une ouverture proximale dans la lumière de fil de guidage suiveur (34A) de sorte que le fil de guidage suiveur (36A) s'étende depuis l'extérieur de l'ouverture d'extrémité proximale, à travers la lumière de fil de guidage suiveur (34A), et hors de l'ouverture d'extrémité distale.

12. Ensemble d'introduction d'extenseur selon la revendication 7, dans lequel le cathéter de vaisseau principal (50) est un cathéter à échange rapide et comprend une ouverture d'extrémité distale dans la lumière de fil de guidage suiveur (53A) et une ouverture d'orifice latérale (53C) sur une surface extérieure du cathéter de vaisseau principal (50), de sorte que le fil de guidage suiveur (36A) s'étende à travers l'ouverture d'orifice latérale (53C) sur la surface extérieure du cathéter de vaisseau principal (50), à travers la lumière de fil de guidage suiveur (53A), et hors de l'ouverture d'extrémité distale du cathéter de vaisseau principal (50), le cathéter de vaisseau principal (50) comprenant, en outre, une fente s'étendant depuis l'ouverture de l'orifice latérale (53C) de sorte que le fil de guidage suiveur (36A) puisse être tiré à travers la fente pendant un échange de cathéter.

13. Ensemble d'introduction d'extenseur selon la revendication 7, dans lequel le cathéter de vaisseau principal (50) est un cathéter sur le fil et comprend une ouverture d'extrémité distale dans la lumière de fil de guidage suiveur (53A) et une ouverture proximale dans la lumière de fil de guidage suiveur (53A) de sorte que le fil de guidage suiveur (34A) s'étende depuis l'extérieur de l'ouverture de l'extrémité proximale, à travers la lumière du fil de guidage suiveur (53A) et hors de l'ouverture de l'extrémité distale.
